# EUROPEAN PATENT APPLICATION

(11) **EP 1 914 321 A2**
(43) Date of publication of application: **23.04.2008**
(21) Application number: 07118003.8
(22) Date of filing: 06.10.2007
(51) Int. Cl.: C12Q 1/68

(54) **Selection procedure by excess ligands**

(30) Priority: 06.10.2006 AR P060104421
(71) Applicant: Radrizzani Helguera, Martin, 25 de Mayo 611, Piso 6°, Of. 2 1009 Buenos Aires (AR); Halitus Instituto Medico S.A., 25 de Mayo 611, Piso 6°, Of. 2 1009 Buenos Aires (AR)
(72) Inventor: Radrizzani Helguera, Martín, 1009, Buenos Aires (AR)
(74) Representative: Elzaburu Marquez, Alberto

(57) **Abstract**

SELECTION PROCEDURE IMPLEMENTED BY EXCESS LIGANDS comprising a stochastic method based on the probability of the existence of a high number of possible ligands against the same target. The objective of this protocol is to provide libraries with concentrations of ligands compatible with the equilibrium concentrations in solution. This invention provides ligands consisting of oligonucleotides and each of them has a sequence-defined structure capable of recognizing the target used for the selection. *The advantage of this method over the other methods is the ability to obtain a large number of specific ligands in only one step and in a few hours.* When proteins are used as targets together with combining oligonucleotide libraries, the ligands obtained are of a nanomolar range affinity and are sensitive to the constitution thereof. Moreover, this method allows for the detection of differential targets of complex mixtures, without need of pure substances.

## Description

### FIELD OF THE INVENTION

This invention consists of a protocol for quickly obtaining large amounts of ligands in a single "in vitro" selection from a combinatorial library. Synthetic chains of the species obtained are capable of specifically identifying the chosen target and therefore may be used for diagnosis, nanotechnology, purification or any other application that requires generating a specific interaction. Therefore, this invention addresses the need to obtain specific ligands in a simple, fast and efficient process against a wide variety and large number of targets.

Protein detection is usually done through the use of poly- and monoclonal antibodies. Since they are proteins, they cannot be used in microarrays, and the cost of obtaining them is steep, since cells or animals are required to produce them. An aptamer is an emerging type of molecule capable of specifically identifying molecule targets such as proteins. These ligands are obtained "in vitro" from combinatorial libraries by means of a procedure called SELEX. Given the complexity and the effort required to obtain such aptamers, only a few hundred of them were obtained after two decades.

### BACKGROUND AND ADVANTAGES OF THE INVENTION

An "In vitro" selection of oligonucleotides with random sequences may offer reagents that bind to a wide variety of substances of high binding affinity and specificity, including small organic molecules, peptides, proteins and macromolecular structures such as viruses or cells (1). These sophisticated kinds of ligands composed of nucleic acids were first generated by means of the SELEX method in 1990 (2) and have ever since become a new alternative to antibodies for identifying targets.

Like the antibodies, the ligands adapt to diagnosis reagents and are capable of detecting and quantifying substances in solution. Given their nature of nucleic acids, these ligands are strong in assays involving microarrays of genomic and proteomic preparations.

The SELEX method, which stands for "Systematic Evolution of Ligands by Exponential Enrichment" constitutes an excellent procedure to produce ligands or aptamers (2). It was developed by Larry Gold's team at the University of Colorado (Gold G, Tueck C, Dec. 1993 US patent 5,270,163) and has since permitted the identification of hundreds of aptamers with high affinity for molecular targets of various sizes.

The SELEX method is an iterative process consisting of four basic steps: (1) preparing the mixture of ligands; (2) selecting (presenting and partitioning); (3) amplifying; and (4) isolating the ligand or aptamer.

In order to prepare the mixture of ligands, a large combinatorial library of nucleic acids is initially generated. The mixture of candidate ligands includes a fixed region flanking a random region composed of random nucleotides. The library contains 10¹⁵ species of oligonucleotides with a single variable sequence which theoretically can adopt a tri-dimensional structure of their own. One of such simple chains may supplement the surface of the molecular target.

As for selection, this stage can be said to have been designed to obtain molecules with high affinity with their target. The presentation of the combinatorial library is done throughout a period of time, selecting those ligands with increased affinity in a partition, and discarding those not bound to the target. Any of the methods used to physically separate bound ligands from unbound ones may be used in this stage of the SELEX protocol.

The amplification stage involves separating bound chains from the target, copying and/or amplifying them using enzymes, generating a new library enriched with those isoforms capable of binding with the selected target. This new combinatorial library is used for a new selection, partition and amplification cycle. These processes are iterative, i.e., they are repeated as many times as necessary until high-affinity ligands are obtained.

Finally, as regards isolating the ligands, we know that after the 5-15 cycles needed to complete the SELEX process, the number of ligands obtained is reduced from 10¹⁵ down to a few species that bind tightly to their target. Molecules so obtained are individually isolated, their sequence is determined and the binding properties are measured and compared. In many cases, the selection is more refined, eliminating nucleotides that do not contribute to the binding and/or structure of the aptamer. The length of polymers generally ranges from 15 to 60 nucleotides.

This technique has produced results against a Wide variety of small molecules including adenosine (3), dopamine or its molecular evolution up to I-tyrosine (4, 5), I-tyroxinamine (6), isoleucine (7) or proteins such as the epidermal growth receptor 3 (8), HIV virus proteins (9-23), hepatitis B and C (24-30), thrombin (31-40), elastase (41, 42), interferon-gamma (43), and pathogenic and cellular prion (44, 45). The SELEX protocol has even been applied to whole tumor cells and a differential target such as tenascin-C (46, 47). Chemical modification and photo-activation to enable the aptamer to bind to the target were carried out as an additional embodiment for use in reactions with antibodies for diagnostic tests (56).

The tools used with proteins during the "genomic" era make SELEX an insufficient protocol to meet current needs, and further efforts have been made to modify the method. One such strategy consists in using as "temporary target" a synthetic peptide from a sequence contained in the chosen protein. After obtaining a library capable of recognizing the peptide, the target is replaced and the library is presented against the whole protein, either in pure state or in a mixture. This strategy allows for making ligands without a need for pure proteins (48-51) (European patent to Santa Coloma TA (EP 1 078 992 A3)). Other efforts have focused in the automation of the SELEX procedure to obtain aptamers against specific targets (52-55). Assays are also performed for use in nanotechnology, diagnosis (57) and selection with complex targets such as cells (47, 58-61). The number of aptamers or nucleic ligands against proteins since 1991 is documented in databases, and as of August 8, 2005, 3498 sequences are stored in http://aptamer.icmb.utexas.edu, from 308 scientific papers. In spite of this enormous potential for development, no reasonable amounts of ligands have been obtained to meet the population's various needs.

The numbers between brackets identify the following documents: 1) Jayasena, S.D., Aptamers: an emerging class of molecules that rival antibodies in diagnostics. Clin Chem, 1999. 45(9): p. 1628-50., 2) Tuerk, C. and L. Gold, Systematic evolution of ligands by exponential enrichment: RNA ligands to bacteriophage T4 DNA polymerase. Science, 1990. 249(4968): p. 505-10., 3) Burke, D.H. and L. Gold, RNA aptamers to the adenosine moiety of S-adenosyl methionine: structural inferences from variations on a theme and the reproducibility of SELEX. Nucleic Acids Res, 1997. 25(10): p. 2020-4.; 4) Mannironi, C., et al., Molecular recognition of amino acids by RNA aptamers: the evolution into an L-tyrosine binder of a dopamine-binding RNA motif. Rna, 2000. 6(4): p. 520-7., 5) Mannironi, C., et al., In vitro selection of dopamine RNA ligands. Biochemistry, 1997. 36(32): p. 9726-34., 6) Marshall Ka Fau - Ellington, A.D. and A.D. Ellington, Training ribozymes to switch. Nat Struct Biol, 1999. 6(11): p. 992-4., 7) Lozupone, C., et al., Selection of the simplest RNA that binds isoleucine. Rna, 2003. 9(11): p. 1315-22., 8) Chen, C.H., et al., Inhibition of heregulin signaling by an aptamer that preferentially binds to the oligomeric form of human epidermal growth factor receptor-3. Proc Natl Acad Sci U S A, 2003. 100(16): p. 9226-31., 9) Tuerk, C. and S. MacDougal-Waugh, In vitro evolution of functional nucleic acids: high-affinity RNA ligands of HIV-1 proteins. Gene, 1993. 137(1): p. 33-9., 10) Giver L Fau - Bartel, D.P., et al., Selection and design of high-affinity RNA ligands for HIV-1 Rev. Gene, 1993. 137(1): p. 19-24., 11) Jensen, K.B., et al., Characterization of an in vitro-selected RNA ligand to the HIV-1 Rev protein. J Mol Biol, 1994. 235(1): p. 237-47., 12) .Allen, P., S. Worland, and L. Gold, Isolation of high-affinity RNA ligands to HIV-1 integrase from a random pool. Virology, 1995. 209(2): p. 327-36., 13) Schneider, D.J., et al., High-affinity ssDNA inhibitors of the reverse transcriptase of type 1 human immunodeficiency virus. Biochemistry, 1995. 34(29): p. 9599-610., 14) .Allen, P., et al., A specific RNA structural motif mediates high affinity binding by the HIV-1 nucleocapsid protein (NCp7). Virology, 1996. 225(2): p. 306-15., 15) Burke, D.H., et al., Bent pseudoknots and novel RNA inhibitors of type 1 human immunodeficiency virus (HIV-1) reverse transcriptase. J Mol Biol, 1996. 264(4): p. 650-66., 16) Chen, H., et al., Inhibitory RNA ligand to reverse transcriptase from feline immunodeficiency virus. Biochemistry, 1996. 35(21): p. 6923-30., 17) Symensma TI Fau - Giver, L., et al., RNA aptamers selected to bind human immunodeficiency virus type 1 Rev in. J Virol, 1996. 70(1): p. 179-87., 18) Berglund, J.A., B. Charpentier, and M. Rosbash, A high affinity binding site for the HIV-1 nucleocapsid protein. Nucleic Acids Res, 1997. 25(5): p. 1042-9.,19) Marozzi, A., et al., In vitro selection of HIV-1 TAR variants by the Tat protein. J Biotechnol, 1998. 61(2): p. 117-28., 20) Andreola, M.L., et al., DNA aptamers selected against the HIV-1 RNase H display in vitro antiviral activity. Biochemistry, 2001. 40(34): p. 10087-94., 21) .Andreola, M.L., et al., HIV-1 integrase and RNase H activities as therapeutic targets. Expert Opin Ther Targets, 2002. 6(4): p. 433-46., 22) Darfeuille, F., et al., 2'-O-methyl-RNA hairpins generate loop-loop complexes and selectively inhibit HIV-1 Tat-mediated transcription. Biochemistry, 2002. 41 (40): p. 12186-92., 23) .Joshi, P. and V.R. Prasad, Potent inhibition of human immunodeficiency virus type 1 replication by template analog reverse transcriptase inhibitors derived by SELEX (systematic evolution of ligands by exponential enrichment). J Virol, 2002. 76(13): p. 6545-57., 24). Ali, N., et al., Human La antigen is required for the hepatitis C virus internal ribosome entry site-mediated translation. J Biol Chem, 2000. 275(36): p. 27531-40., 25). Vo, N.V., J.W. Oh, and M.M. Lai, Identification of RNA ligands that bind hepatitis C virus polymerase selectively and inhibit its RNA synthesis from the natural viral RNA templates. Virology, 2003. 307(2): p. 301-16., 26). Anwar, A., et al., Demonstration of functional requirement of polypyrimidine tract-binding protein by SELEX RNA during hepatitis C virus internal ribosome entry site-mediated translation initiation. J Biol Chem, 2000. 275(44): p. 34231-5., 27) Bellecave, P., et al., Selection of DNA aptamers that bind the RNA-dependent RNA polymerase of hepatitis C virus and inhibit viral RNA synthesis in vitro. Oligonucleotides, 2003. 13(6): p. 455-63., 28) Biroccio, A., et al., Selection of RNA aptamers that are specific and high-affinity ligands of the hepatitis C virus RNA-dependent RNA polymerase. J Virol, 2002. 76(8): p. 3688-96., 29) Fukuda, K., et al., Isolation and characterization of RNA aptamers specific for the hepatitis C virus nonstructural protein 3 protease. Eur J Biochem, 2000. 267(12): p. 3685-94., 30) Hu, K., J. Beck, and M. Nassal, SELEX-derived aptamers of the duck hepatitis B virus RNA encapsidation signal distinguish critical and non-critical residues for productive initiation of reverse transcription. Nucleic Acids Res, 2004. 32(14): p. 4377-89., 31) Liu, X., et al., Screening of functional antidotes of RNA aptamers against bovine thrombin. FEBS Lett, 2004. 562(1-3): p. 125-8., 32) Bock, L.C., et al., Selection of single-stranded DNA molecules that bind and inhibit human thrombin. Nature, 1992. 355(6360): p. 564-6., 33) Griffin, L.C., J.J. Toole, and L.L. Leung, The discovery and characterization of a novel nucleotide-based thrombin inhibitor. Gene, 1993. 137(1): p. 25-31., 34) Kubik, M.F., et al., High-affinity RNA ligands to human alpha-thrombin. Nucleic Acids Res, 1994. 22(13): p. 2619-26., 35) Tasset, D.M., M.F. Kubik, and W. Steiner, Oligonucleotide inhibitors of human thrombin that bind distinct epitopes. J Mol Biol, 1997. 272(5): p. 688-98., 36) Jenison, R.D., et al., Oligonucleotide inhibitors of P-selectin-dependent neutrophil-platelet adhesion. Antisense Nucleic Acid Drug Dev, 1998. 8(4): p. 265-79., 37) Drolet, D.W., et al., A high throughput platform for systematic evolution of ligands by exponential enrichment (SELEX). Comb Chem High Throughput Screen, 1999. 2(5): p. 271-8., 38) White, R., et al., Generation of species cross-reactive aptamers using "toggle" SELEX. Mol Ther, 2001. 4(6): p. 567-73., 39) .Spiridonova, V., et al., A comparative thermodynamic study for both natural and artificial RNA/DNA-protein binary complexes. Bio-electrochemistry, 2002. 56(1-2): p. 95-7., 40) Liu, X., et al., RNA aptamers specific for bovine thrombin. J Mol Recognit, 2003. 16(1): p. 23-7., 41) Charlton, J., G.P. Kirschenheuter, and D. Smith, Highly potent irreversible inhibitors of neutrophil elastase generated by selection from a randomized DNA-valine phosphonate library. Biochemistry, 1997. 36(10): p. 3018-26., 42) Bless, N.M., et al., Protective effects of an aptamer inhibitor of neutrophil elastase in lung inflammatory injury. Curr Biol, 1997. 7(11): p. 877-80., 43) Kubik, M.F., et al., Isolation and characterization of 2'-fluoro-, 2'-amino-, and 2'-fluoro-/amino-modified RNA ligands to human IFN-gamma that inhibit receptor binding. J lmmunol, 1997. 159(1): p. 259-67., 44) Takemura, K., et al., DNA aptamers that bind to PrP(C) and not PrP(Sc) show sequence and structure specificity. Exp Biol Med (Maywood), 2006. 231 (2): p. 204-14., 45) Begic, L., et al., (New findings on the diagnosis and therapy of prion diseases). Med Arh, 2002. 56(5-6): p. 305-11., 46) Hicke, B.J., et al., Tenascin-C aptamers are generated using tumor cells and purified protein. J Biol Chem, 2001. 276(52): p. 48644-54., 47) Daniels, D.A., et al., A tenascin-C aptamer identified by tumor cell SELEX: systematic evolution of ligands by exponential enrichment. Proc Natl Acad Sci U S A, 2003. 100(26): p. 15416-21., 48) Bianchini, M., et al., Specific oligobodies against ERK-2 that recognize both the native and the denatured state of the protein. J Immunol Methods, 2001. 252(1-2): p. 191-7., 49) Radrizzani, M., et al., Development of monoclonal oligobodies and chemically synthesized oligobodies. Medicina (B Aires), 2000. 60 Suppl 2: p. 55-60., 50) Radrizzani M Fau - Broccardo, M., et al., Oligobodies: bench made synthetic antibodies. Medicina (B Aires), 1999. 59(6): p. 753-8., 51). Zhang Xm Fau - Li, Q., et al., Specific oligobodies against human brain acetylcholinesterase. Brain Res, 2003. 989(2): p. 147-51., 52) Roulet, E., et al., High-throughput SELEX SAGE method for quantitative modeling of transcription-factor binding sites. Nat Biotechnol, 2002. 20(8): p. 831-5., 53) Eulberg, D., et al., Development of an automated in vitro selection protocol to obtain RNA-based aptamers: identification of a biostable substance P antagonist. Nucleic Acids Res, 2005. 33(4): p. e45., 54) Zhang, H., et al., Automated in vitro selection to obtain functional oligonucleotides. Nucleic Acids Symp Ser, 2000(44): p. 219-20.; 55) Hybarger, G., et al., A microfluidic SELEX prototype. Anal Bioanal Chem, 2006. 384(1): p. 191-8.; 56) Brody, E.N., et al., The use of aptamers in large arrays for molecular diagnostics. Mol Diagn, 1999. 4(4): p. 381-8., 57) Herr, J.K., et al., Aptamer-conjugated nanoparticles for selective collection and detection of cancer cells. Anal Chem, 2006. 78(9): p. 2918-24., 58) Wang, C., et al., Single-stranded DNA aptamers that bind differentiated but not parental cells: subtractive systematic evolution of ligands by exponential enrichment. J Biotechnol, 2003. 102(1): p. 15-22., 59) Blank, M., et al., Systematic evolution of a DNA aptamer binding to rat brain tumor microvessels. selective targeting of endothelial regulatory protein pigpen. J Biol Chem, 2001. 276(19): p. 16464-8., 60) Morris, K.N., et al., High affinity ligands from in vitro selection: complex targets. Proc Natl Acad Sci U S A, 1998. 95(6): p. 2902-7., 61) Fitter, S. and R. James, Deconvolution of a complex target using DNA aptamers. J Biol Chem, 2005. 280(40): p. 34193-201.

### PURPOSE OF THIS INVENTION

The purpose of the procedure under this invention, called SIMPLEX, it to obtain ligands that specifically recognize their target-receptor. The acronym derives from the Spanish name "Selección Implementada por Ligandos en Exceso" (Selection Procedure by Excess Ligands), which refers to the improved selection of ligands as a result of increasing the number of ligands during presentation with the target. Under this procedure, some species from the combinatorial library are amplified stochastically so that they reach the required concentrations in binding assays. The number of ligand molecules bound in one partition is increased and the species obtained have affinity and specificity against their targets without any other step being necessary. These libraries are capable of recognizing complex target mixtures, and if they are in excess the bound ligands can be separated from the rest. The remaining ligands are capable of recognizing different targets, only present in a second mixture. The ligands obtained by the SIMPLEX method compared to other procedures have lower affinity and are sensitive to the constitution of the target. Like aptamers, these ligands are appropriate to detect and quantify their targets, generate and produce microarrays for studying proteins, characterizing complex mixtures and specifically inhibiting enzymes, with applications in diagnosis and nanotechnology alike.

The primary advantage of this protocol over the other methods is that it can be done in hours, without cycles, producing a large number of ligands against the chosen target.

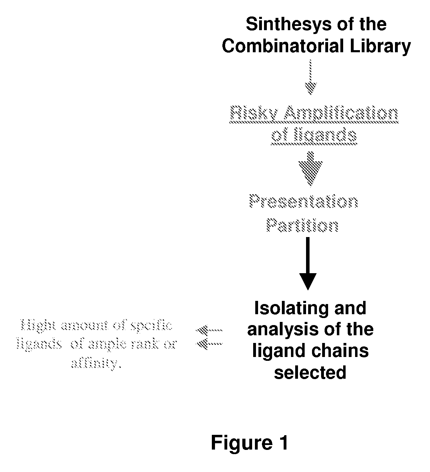

The SIMPLEX protocol is built upon the principle of "equilibrium of ligands and receptor targets in solution". The process consists of four basic steps, to wit: (1) Preparing the combinatorial library: just like other methods, the ligands composing the combinatorial oligonucleotide library are produced by chemical synthesis. The great variability of ligands depends on the length of the variable zone and is physically limited to 10¹⁴-10¹⁵ different species in solution. 2) Amplifying the library: unlike any other protocol, preparing a SIMPLEX library sacrifices variability in favor of allowing for a number of species to reach the necessary concentration to attain balance in solution. For that reason, synthesized forms are subjected to an exponential amplification process. That is, each ligand which is amplified has higher chances of being amplified again, thus increasing the number of some species randomly. In this way, each library increases in the selection the number and quality of binding species. 3) Presenting and partitioning the ligands: amplification allows for species to reach conditions of balance in solution with their potential receptor target. Targets may be presented either in solution or on a solid support. The presentation of the amplified library in solution must remain in contact for as long as necessary to reach the binding balance, which can range from minutes to hours. Any known partitioning system may be used to separate bound chains from those kept in solution; and finally, 4) Isolating the ligands: separated chains are analyzed one by one to know the sequence of oligonucleotides. The characteristics of each chain are measured and compared in order to select the best ligands. Figure 1 illustrates the sequence mentioned in the above paragraph.

Products obtained have differing characteristics. The most prominent difference is the affinity of ligands. The SIMPLEX method selects ligands with affinity comparable to that of antibodies, but up to 10 times smaller than ligands obtained through other protocols. The advantage of this method is that its selection specter covers numerous forms not selected by the other protocols. This advantage does away with the need for more selection-partition-amplification cycles.

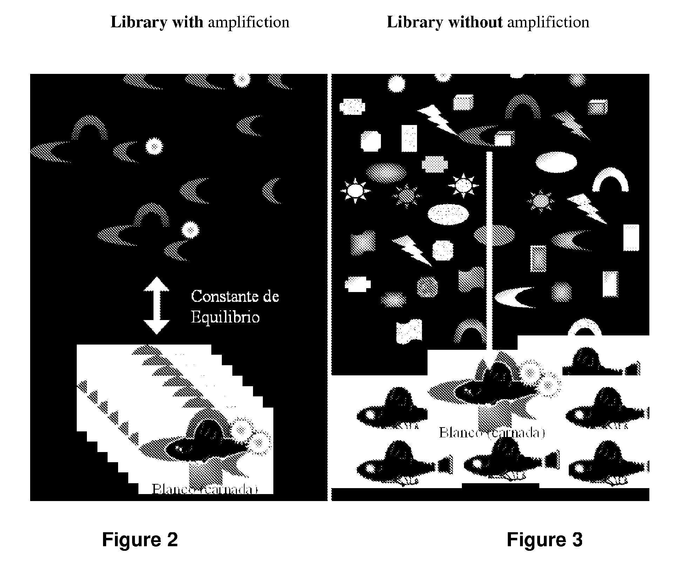

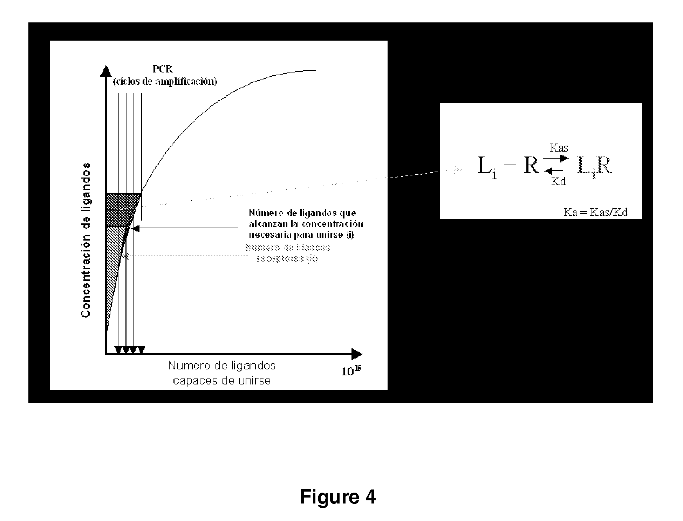

The model upon which this protocol is built is based on the following: (1) The number of specific ligands must increase. Figures 2 and 3 show the impact of amplifying the library on the number of bound ligands. As a result of the selection, many molecules of one kind are selected in a single step. The increase in the concentration of a ligand before partition allows it to be selected in the SIMPLEX protocol; (2) the types of ligands presented in the selection can outnumber the targets presented. The combinatorial library is set up so as to attain a balance between the concentration of each species and the likelihood of having species that recognize the chosen target. Figure 4 shows the relation between the concentration in the selection and the each species' likelihood of recognizing the target. If the minimum concentration is not reached, many of these species might not be selected. When the molecules of certain target are outnumbered by the ligands, the various species compete against each other, favoring the selection of the best ligands.

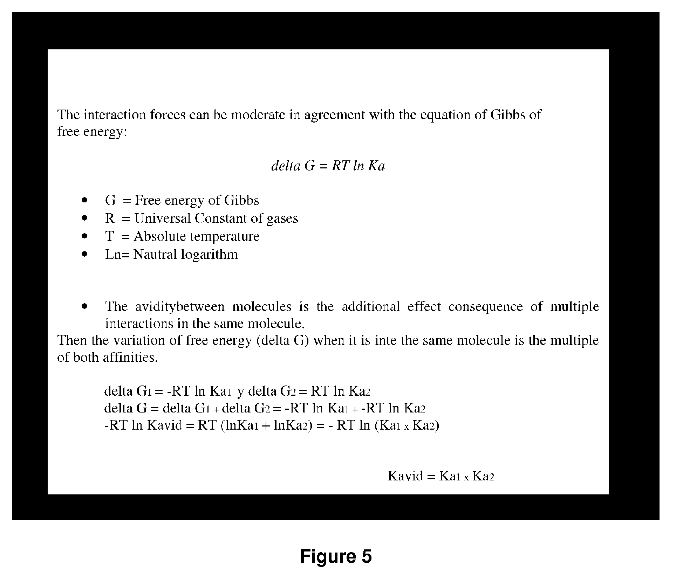

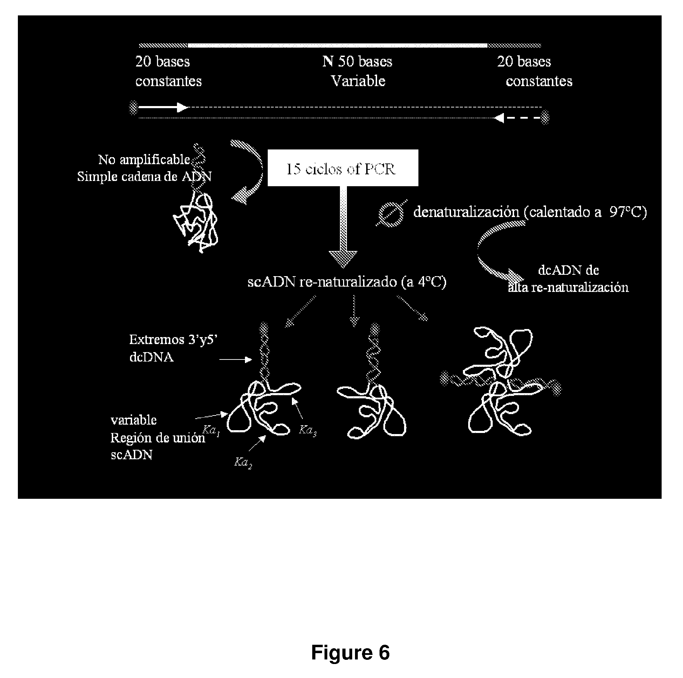

In order to build a library, the following considerations were taken into account. The likelihood of finding a species with specificity towards its target depends on the number of species, that is, the length of the variable region. For example, a species may bind in only one high-affinity site and another may do so in several high-affinity sites (figure 5). If the procedure requires increasing the number of copies of one species, the amplification prior to selection assures that the species participating in the selection will be substrate for enzymes (figure 6).

### EXAMPLES OF APPLICATION OF THE SIMPLEX PROTOCOL: DNA LIGANDS AND PROTEIN TARGETS

For the application of the SIMPLEX protocol, peptides and proteins were chosen as target/receptor and single-stranded DNA oligonucleotides are used as ligands.

The combinatorial library is produced by the chemical synthesis of deoxyribonucleotide polymers. It is designed with two fixed regions flanking a random region of approximately 50 oligonucleotides. Fixed regions were designed with inverse and complementary sequences, forming a double helix and structuring the random region.

The chosen amplification method is the polymerase chain reaction (PCR). This reaction requires at least one primer with reverse sequences and sequences which are complementary to the fixed regions and the synthetic library as a reaction template. When both ends of the library are reverse and complementary, the chain adopts a stable constitution in solution. During amplification, chains whose structure is too stable will not be amplified by polymerase. On the other hand, amplified chains will result in double-stranded products which, when separated, originate two simple strands which are bilaterally symmetrical, doubling the possibilities of the obtained ligands (figure 6). Amplifying the library is a key step to obtain a large amount of ligands in a single partition. Given a number of receptor targets, there are two defined variables to reach a library's maximum potential. The first is the number of forms to be amplified, that is, the starting synthetic library number as shown in example 1.1. The second variable is the number of cycles needed for each library to reach a substantial amount of specific ligands. The number of cycles needed to saturate receptor proteins with that concentration of ligands is determined as shown in example 1.2. The example shows the relation between the increase in the number of amplification cycles and the number of ligands obtained in one partition.

Presentation of target-receptors: the 20 nanomoles of peptide are presented on a solid support against a theoretical 30-picomole concentration for each ligand, that is, if the PCR amplified all chains equally for at least 15 cycles. It is in these conditions that the bound ligands outnumber the targets, resulting in a stable maximum number of ligands as shown in example 1.2. The limiting factor is the number of targets and there is a direct relation between the number of ligands obtained and the amounts of target used with a single library concentration (example 1.3).

Isolating and characterizing ligands: the protocol applies to peptide and protein targets with synthetic combinatorial libraries. The DNA ligands obtained in these examples are termed "oligo-antibodies" (Ob) because of their similarity with classical protein antibodies in the recognition of peptides and proteins. The ligand chains obtained after partition are converted to double chain and cloned in bacterial expression vectors. The chosen clones are studied using plasmid as a template source for biochemical amplification of PCR and later used to study their properties (example 2). The selected chains are chemically synthesized for use in detection assays where the specificity of each reagent is shown (examples 2 and 3).

Exclusion of ligands for detecting differential targets in complex mixtures. The protocol is also applied to complex targets as mixtures of different proteins, for example a tissue such as the murine cerebellum. The amplified library is capable of recognizing a large number of proteins, and these are not detected during control when pre-incubated with the used proteins. When molecules are isolated, special care must be taken not to over-amplify the mixture since, with a PCR of 25 cycles, variability is lost as shown in example 4.1. When the targets are very similar, bound units are scarce and the target detection variability is lost in the exponential amplification. Such is the case of proteins from a line of tumor cells in a murine lung. For the subtraction, ligands could be amplified after 22 PCR cycles and only a single different 40 kDa band was detected, losing variability in the number of targets probably as a result of exponential amplification (Example 4.2). The combinatorial library is adjusted, subtracted and the free ligands recognize a large number of differential targets, such as proteins with temporary expression during the development of the murine cerebellum (example 4.3).

### CHARACTERISTICS OF THE PRODUCTS OBTAINED BY THE SIMPLEX PROCESS.

Pages 54 to 68 describe the sequences of the libraries used in the "selection procedure by excess ligands", applied to fifteen peptides and proteins. They also describe the 59 sequences obtained, with proven interaction with the 15 targets used.

The examples presented show that oligo-antibodies have affinity with their target-receptor of about 100 nM for four different proteins: lysozyme, Glutation-S-Transferase protein, Acidic Nuclear phosphoprotein 32 member e (Anp32e/Cpd1) and the PTEN protein. Ligand mixtures are capable of recognizing twice as many peptides in solution as a single species, showing that one peptide can bind two ligands. The interpretation of the result is that there is one ligand for every 7 amino-acids or, potentially, two different constitutions of the peptides presented (example 2.3). Ligands are capable of blocking enzymes' activities. For example, the oligo-antibody blocks the purified protein Anp32e's inhibiting the PP2A phosphatase (example 3.19). Oligo-antibodies also recognize pure targets in solution or in mixtures as shown in the gel retardation assays (example 3.15). These ligands are capable of recognizing their targets in complex mixtures of proteins in assays for detecting cerebellum proteins separated by electrophoresis and transferred to nitrocellulose membranes (example 3.1). They also recognize the recombinant protein in bacteria homogenates and detect proteins in histological sections with peroxidase staining (example 3.10) or in electronic microscopic assays (example 3.4). Oligo-antibodies were also able to purify the target contained in complex mixtures (Example 3.3) and, with classical antibodies, confirm the identity of the target (example 3.4-3.6). Target presentation defines the binding sites and therefore recognizing the target also depends on its constitution (examples 3.7, 3.8 and 3.13). The method's sensitivity allows for obtaining ligands capable of differentiating the change of only 1 out of 14 amino-acids contained in the target peptide (examples 3.7 and 3.12). Oligo-antibodies obtained by the SIMPLEX protocol are capable of recognizing their target even when the ligand is contained in a polymer (example 3.9). Proteins too can be detected in sections without cross-reactions between oligo-antibodies by using fluorescence (examples 3.15) Example 3.17 illustrates an oligo-antibody obtained from an enriched mixture of the protein chosen as target, without a need for purification. This oligo-antibody is capable of recognizing the protein in a native state, in solution, in sections and denatured in the nitrocellulose membrane.

All these examples position the SIMPLEX protocol as a powerful tool for forming DNA-peptide/protein interaction pairs quickly and efficiently. The nature of the DNA structure together with its specificity in recognizing its targets makes these ligands a complement to classical antibodies for studying, detecting or applying toxic or non-immunogenic proteins.

### LIBRARY WITH AMPLIFICATION-LIBRARY WITHOUT AMPLIFICATION MODEL (FIGURES 2 AND 3)

The number of ligands obtained is too small relative the non-specific binding of other chains and thus more selection and amplification cycles are required before collecting and identifying the ligands obtained. The SIMPLEX protocol prefers to amplify production of potential ligands and sacrifice variability to reach the target-ligand balance in solution. If so, at least one species will be selected, but in a large number of copies and in only one selection step.

The SIMPLEX method was developed under the concept of balance of masses in solution (Figure 4).
1. The ligand concentration (L) must reach a minimum level. The minimum concentration level depends on each ligand species' affinity constant (Li.)
2. The number of ligands is limited by the number of sites in the chosen receptor (R) during partition (light green).

The number of ligand species characterizes the construction of each library, and the likelihood of finding a candidate will depend on its amplification. The combinatorial library is amplified, and if a given ligand species reaches the minimum concentration, it will occupy a number of receptors. If several species do, the number of bound molecules rises in the partition until it outnumbers the targets. In such event, each ligand is selected over non-specific binds. This amplification is essential for specific ligands with lower affinity to be presented and selected, also increasing the number of ligand species capable of binding. This does away with the need to repeat amplification and selection processes, unlike other protocols which do require repetition to select a small number of ligands with high affinity for the bind in a random or non-specific fashion.

### CONSIDERATIONS FOR EXPERIMENTAL DESIGN OF LIBRARIES

Figure 5 shows that the strategy of the SIMPLEX protocol seeks to incorporate the largest number of potential ligands during the selection process. The length of the random region is determined according to the number of possible binding sites in each ligand molecule and the equipment's synthesis efficiency. With lengths of 50 nucleotides, there would be 10³⁰ different molecules and the maximum number of molecules reached by chemical synthesis is 10¹⁶. Given sites with a length of 15 to 20 nucleotides, there would be 2 to 3 sites to bind the receptor in each ligand molecule. The formula illustrated in the diagram shows how affinity constants contribute to a single molecule. For example, two binding sites of 10⁻³ each would produce an interaction of around 10⁻⁶ or even micro molar. This allows for the expansion of the universe of potential ligands for a given receptor and, additionally, to be sensitive to distance between binding sites.

As shown in figure 6, the central area is the random region (white), flanked by a constant sequence region (grey) and a reverse and complementary region on the other end (dark grey). This structure allows us to polarize the presentation of the 50-nucleotide random region with its potential binding sites. The length allows for more than one binding site (Kan), as proposed in the above-mentioned model.

### RESULTS OF APPLYING THE SIMPLEX METHOD

### EXAMPLE 1.1

### AMPLIFICATION OF THE COMBINATORIAL LIBRARY

In this example the following nucleotides were used:
Primer 1: 5'-CGGAATTCCGAGCGTGGGCGT-3' 21 mer
Primer 2: 5'-CTCGAGCGTGGGCGTA-3' 16 mer 
Template: 5'-CGGAATTCCGAGCGTGGGCGT (N)50
TACGCCCACGCTCGAG-3' 87 mer
Primers 1 and 2: final concentration of 1 µM.
Template: final concentration of 1 µM or 200 nM, as shown in the figure.
Taq polymerase: final concentration of 0.05 U/µl.

Reaction buffer: final concentration of KCI 50 mM, Tris-HCl 10 mM pH 9.0 at 25 °C, MgCl₂ 1.5 mM, Triton X-100 0.1% and nucleotides triphosphate or dNTPs 100 µM.

Amplification cycles are: 94°C by 30", 58 °C by 30", 72°C by 10"

The efficiency of the combinatorial library amplification with the *termophilus aquaticus* polymerase was measured using real-time PCR and SYBRGreen as fluorescent to measure the increase in synthesized chains. The results showed an efficiency of e=1.7.

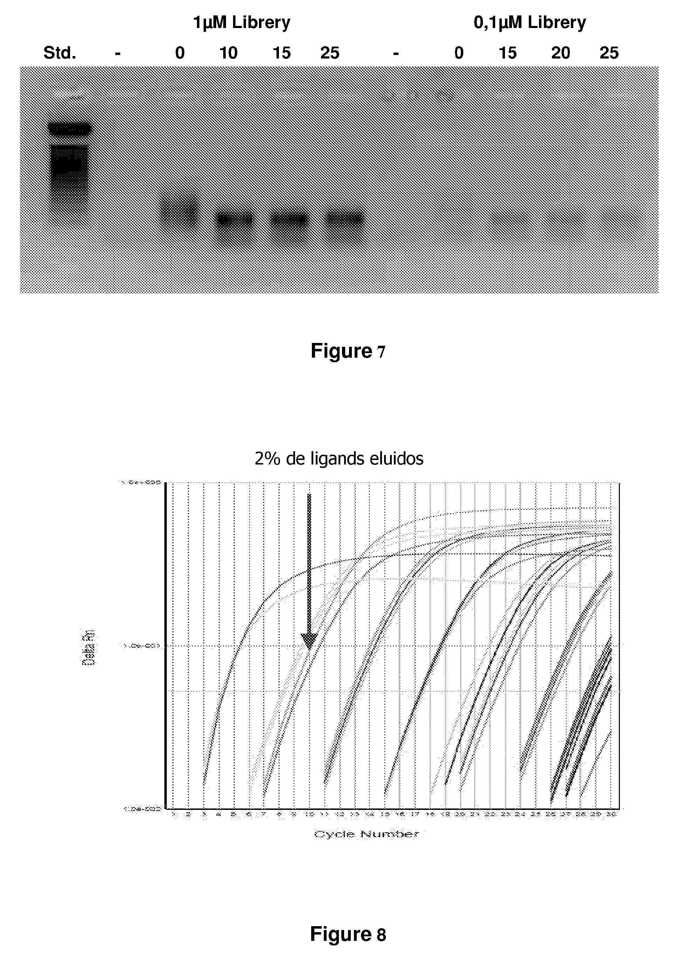

Figure 7 shows the amount of DNA separated by electrophoresis in a 3% agarose gel and viewed with ethidium bromide. The first strand shows 5 micrograms of DNA in 50-nucleotide long polymers (50bp). Differences can be seen in the mobility and intensity of unamplified bands (0) of that product amplified by 15, 20 or 25 cycles (15, 20, 25). There are no amplification products if template is added.

### PRESENTATION OF THE AMPLIFIED LIBRARY WITH ITS TARGET

Presentation of the library with the target is carried out in a mixture consisting of 300 µl of 25-cycle PCR products and 600 µl of phosphate buffer with 1% bovine serum albumin (PBS-BSA 1%) and incubated with shaking overnight at 4ºC. The membrane is washed out, ligands are eluted with pH change for under two minutes using 150 mili-normal sodium hydroxide (0.15N NaOH). Out of the resulting 50 µl, 10 are used for the real-time PCR with SYBRGreen I, (1:10,000).
**0,2nM** library: **10 µl eluted**
   - B6=: - control
   - B5: 2 µl (0) library (no PCR)
   - B4: library 15 cycles
   - B3: library 20 cycles
   - B2: library 25 cycles
**0,2 µM** library: **40 µl eluted**
   - C6=: - control
   - C5: 2 µl (0) library (no PCR)
   - **C4**: library **15** cycles
   - C3: library 20 cycles
   - C2: library 25 cycles
**1 µM** library: **10 µl eluted**
   - B11: - control
   - B10: 20 µl (0) library (no PCR)
   - B9: library 15 cycles
   - B8: library 20 cycles
   - B7: library 25 cycles
**1 µM** library: **40 µl eluted**
   - C10: 20 µl (0) library (no PCR)
   - C9: library 15 cycles
   - C8: library 20 cycles
   - C7: library 25 cycles

### PRESENTATION OF THE SELECTED TARGET: PEPTIDES AND PROTEINS

Approximately 20 µg of peptide are bound to 0.2 cm² of nitrocellulose membrane, leaving 5x 10¹³ molecules or its equivalent of 20 nanomoles of peptides fixed to the support. When proteins are used, the amount exposed is 10 to 20 times smaller in concentration. The buffer chosen for presentation for these experiments is saline phosphate (PO₄⁼ 50 mM, NaCl 150 mM pH: 7.4), and albumin is used to block non-specific bounds. Incubations are carried out in shaking during three hours at room temperature or overnight at 4ºC. After incubation, membranes are washed out with 1% PBS-BSA and further three times with PBS for 15 minutes.

### QUANTIFICATION CURVE:

With the four specified parameters, the next step was to obtain oligo-antibodies against a peptide contained in the sequence of the PTEN protein. In this case, the volume of the library used is doubled (600 µl PCR library - 5 min 95ºC - in 1200 µl PBS/ 1% BSA at 4ºC) and incubated overnight with shaking at 4ºC. The partition was done during the membrane washout (PBS, 45 minutes with shaking); the ligands that remained bound were separated from the targets through heat, and one portion is used to quantify by real-time PCR.

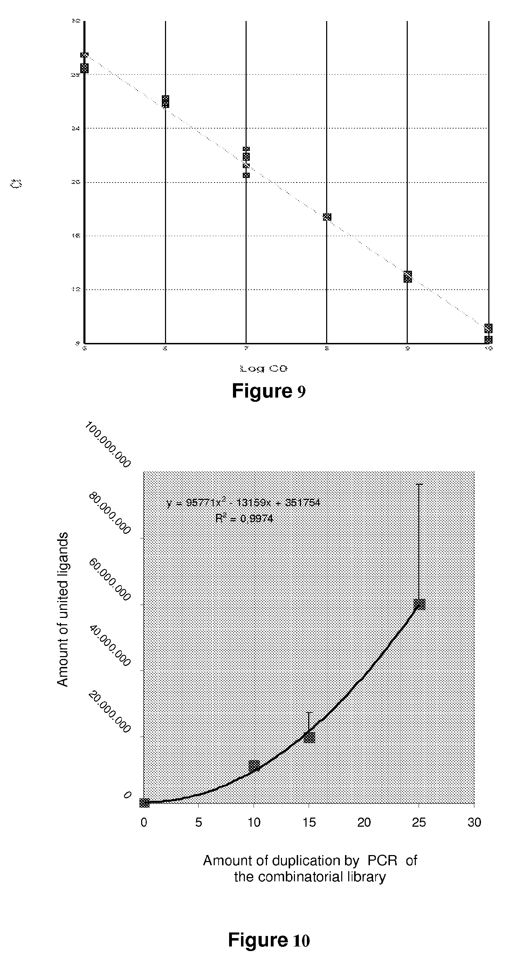

The fluorescence variation is compared in thirty cycles, and the combinatorial library is used in different dilutions to build the standard curve. The estimate number of ligands is 10¹² copies, a similar number to that of the targets presented for the selection (Figure 8).

### CT VS. LOG OF THE NUMBER OF COPIES

The curve represents the ratio between the initial amount of template and the number of cycles needed for the fluorescence variation to reach detectable levels (Ct). Reproducibility can be seen in the low dispersion in the cycles. The curve deviation with respect to the ideal value of 2 copies per PCR cycle does not reference the efficiency of polymerase with the primers and template system. According to such deviation, the efficiency of polymerase is estimated at approximately at 1.7.

The extrapolation of the Ct obtained in the specimen with the standard curve shows 10¹³ bound ligands obtained against the PTEN peptide (Figure 9).

### EXAMPLE 1.2

### AMPLIFICATION OF THE COMBINATORIAL OLIGONUCLEOTIDE LIBRARY

Firstly, the library is synthesized using individual load of each nucleotide so that each base reaches the same likelihood of incorporating into the library. For the starting library amplification, an initial concentration of around 1 micromole containing 6 x 10¹⁴ out of the 10³⁰ possible species is used. Then, after 15 amplification cycles with polymerase, there should be 30,000 copies of each species, reaching an approximate concentration of 10⁻¹² M, that is, in the picomolar range.

Figure 7 shows a 3% agarose gel where DNA was separated by size and is viewed by UV-induced fluorescence of ethidium bromide stain intercalated into the double strand. In this example, the following oligonucleotides were used:
Primer: 5'- CGACGTCGGCGCCCCTTCCT-3' 
Template: 5'- CGACGTCGGCGCCCCTTCCT
(N)50AGGAAGGGGCGCCGACGTCG-3'
Template concentration: 1 µM
Primer, oligo 1: 1 uM.
Template: Final concentration: 100 nM.
Nucleotides Deoxy-Nucleotides-Triphosphates or dNTPs: 100 µM
Taq polimerase: final concentration 0.05 U/µl.
Reaction buffer: KCI 50 mM, Tris-HCl 10 mM pH 9.0 at 25 °C,
MgCl₂ 1.5 mM, Triton X-100 0,1%.

Amplification protocol consists of 25 polymerase reaction cycles (denaturation: 30 sec. at 94ºC, hybridization: 30 sec. at 55ºC, elongation: 10 sec. at 72ºC) in a final volume of 100 µl.

### EFFECT OF LIGAND AMPLIFICATION ON THE NUMBER OF LIGANDS BOUND TO THE TARGET

This example shows the requirement of library amplification to increase the number of ligands bound to the target. This example is carried out using four pure replicas of GST-PTEN protein bound to 0.06 cm² of nitrocellulose membrane. These replicas are made taking 100 microliters of library in the PCR mixture with the chains opened by heating (5 min at 95ºC) and 100 microliters of PBS at 4ºC, where they are mixed and incubated overnight with the membrane in shaking. Unbound ligands are rinsed and bound ligands are separated for quantification using real-time PCR.

The graph in Figure 10 shows the amount of bound ligands in relation to the cycles applied to the combinatorial library. Each point represents the average of four individual experiments and the number of cycles used on each library is shown in the abscissas. The average of ligands (squares, number of bound ligands) and their variation (the bar shows standard deviation) are adjusted to a polynomial equation of the second degree (black curve), whose parameters of which are shown within the graph.

The same effect can be observed within a single initial combinatorial library, subjected to amplifications by PCR. Three PCR tubes containing 1 µM of combinatorial library. Taking 33 microliters from each PCR tube, the 100 µM mix is incubated with the membrane containing PTEN. Assayed amplifications correspond to 0, 10 and 25 amplification cycles with Taq polymerase. The correlation between the number of cycles and the amount of bound ligands corresponds with a polynomial curve of the second degree with a much lower data dispersion. This fact shows that the variability in the number of ligands lies at the initial take of the combinatorial library used as template rather than at the treatment with polymerase.

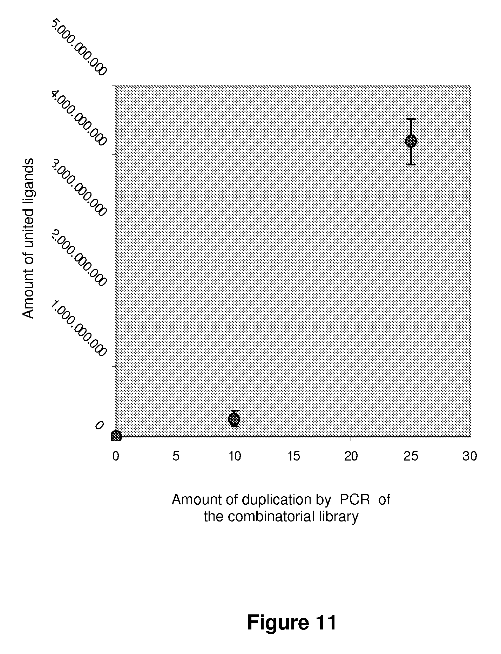

The graph in figure 11 shows the amount of bound ligands in relation with the cycles applied to a single combinatorial library. Each point represents the average of three individual measurements and their correlation with the number of cycles performed on each library. The average of ligands (squares, number of bound ligands) and their variation (the bar measures standard deviation) with a substantial difference (P>0.000005).

### AFFINITY AND SPECIFICITY OF BOUND LIGANDS

Knowing that amplified combinatorial libraries increase the number of ligands, it is necessary to know whether these ligands are specific against the selected target. To analyze ligand specificity and affinity, a nitrocellulose filter retention assay is used. This experiment used ligands obtained with the best and worst selection attained from libraries amplified 25 and 10 PCR cycles. Specificity was observed in ligands obtained with libraries amplified 25 cycles, and little specificity was observed in ligands obtained with 10 cycles. The results show that amplification of species represented in a combinatorial library is necessary to obtain a large number of ligands with specificity and affinity for their target in a single selection.

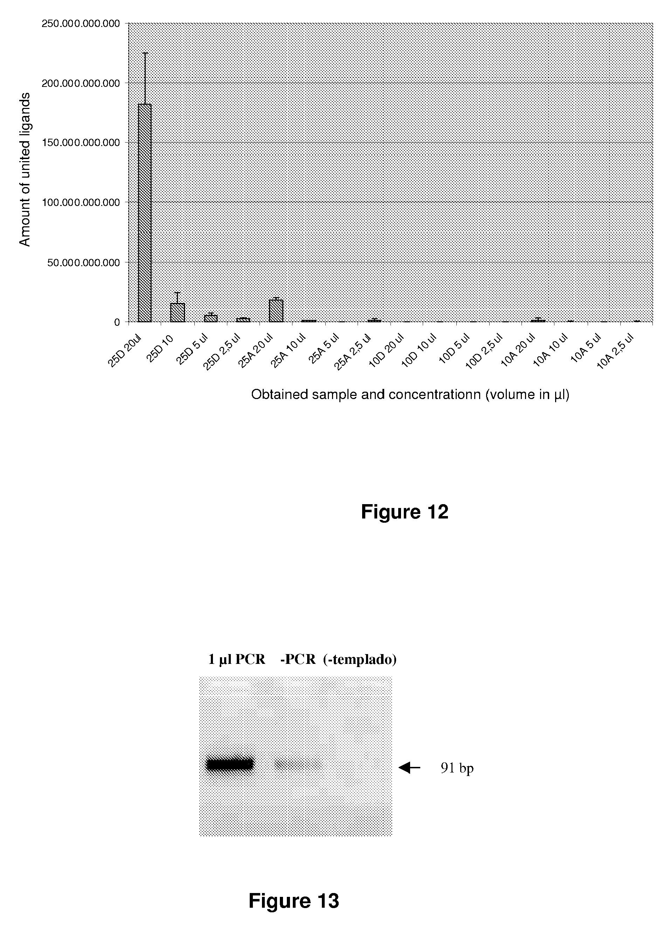

The graph in figure 12 shows that the number of ligands bound to a fixed amount of GST-PTEN protein depends on concentration. Two micrograms of GST-PTEN (3.63 picomoles) are incubated with different concentrations of single-stranded DNA ligands in a final volume of 40 microlitres (90nM of target). Incubation is performed with 500 (20 ul), 250 (10 ul), 125 (5 ul) and 62.5 nM (2.5 ul) of the mixture of ligands amplified by PCR. Chains are separated in a nitrocellulose where the protein is retained with the ligands bound to them. Ligands are then separated from the nitrocellulose and quantified by real-time PCR.

### EXAMPLE 1.3

### QUANTIFICATION OF BOUND LIGANDS

This example shows the calibration of the SIMPLEX combinatorial library designed against peptides and proteins. For example, a 14-amino-acid peptide is used blocking the membrane as initial target, which is used in equal surface and number against different amplifications of a single library. The library used is that from example 1.1, and the target is 20 nanomoles of peptide VLDNCLCVNGEIE (Cpd1b). The library is amplified and 300 µl of PCR products heated to 95ºC during 5 minutes and cooled down with two volumes of 5% PBS-BSA to 4ºC to obtain single-stranded ligands. The membrane is incubated with the library at 4ºC overnight with shaking and washed out three times with PBS, and ligands are separated by heat. Quantification is done through SYBRGreen® (1:10,000), which is intercalated between the fluorescent bases, giving a measure of the amount of PCR product, and ROX as reference fluorescent. The graph shows the increase in the amount of initial template obtained from the selection with different library amplifications (the number of PCR cycles is stated in the squares, and the growth curve is shown in the graph). The amount of ligands obtained is proportional to the number of library amplification cycles. Saturation of receptor targets becomes evident when the total number of ligands obtained from libraries amplified more than 15 cycles remains unchanged.

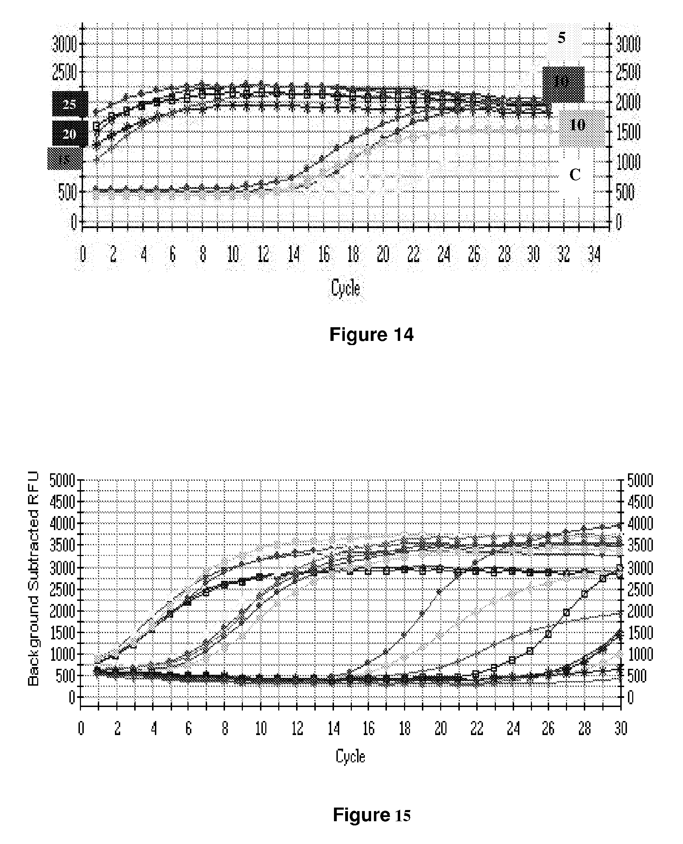

Figures 13 and 14 show that the number of ligands obtained matches the library amplification cycles. After 15 cycles, the number of ligands is determined by the amount of receptor target presented, which is outnumbered by ligands.

### EXAMPLE 1.4

### RELIANCE ON THE NUMBER OF PRESENTED TARGETS: EXCESS OF LIGANDS

Bound ligands rely on the amount of target presented. Measurements were performed using libraries amplified with 25 PCR cycles and presented against the Huntingtin peptide paired with albumin and bound to nitrocellulose. Membrane-free spaces are blocked with bovine albumin, also included in the presentation with the library. Elusion is done by changing the pH using sodium hydroxide (0.15 M NaOH, under 90 sec.) to normalize ligand retrieval. The number of ligands obtained is reflected in the number of cycles needed to detect the amplification of PCR products.

### QUANTIFICATION OF LIGANDS OBTAINED USING DECREASING AMOUNTS OF TARGETS

Figure 15 shows the reliance on the amount of target presented in the selection. 1% of the elusion is used to compare the numbers of ligands obtained against a peptide contained in Huntingtin. One microgram or 200 nanograms of peptide are used as target to show reproducibility in the number of ligands obtained and their relation with the limiting amount of the presented receptor. This relation is also present from 60ng, 50ng, 40ng, 30ng, 20ng (x2). The bottom obtained with albumin exceeds the 10ng peptide presentation in more than 28 cycles.

### EXAMPLE 2.1

### PROPERTIES OF THE PRODUCTS OBTAINED BY THE SIMPLEX PROCEDURE OLIGO-ANTIBODIES: SINGLE-STRAND AND DOUBLE-HELIX STRUCTURE

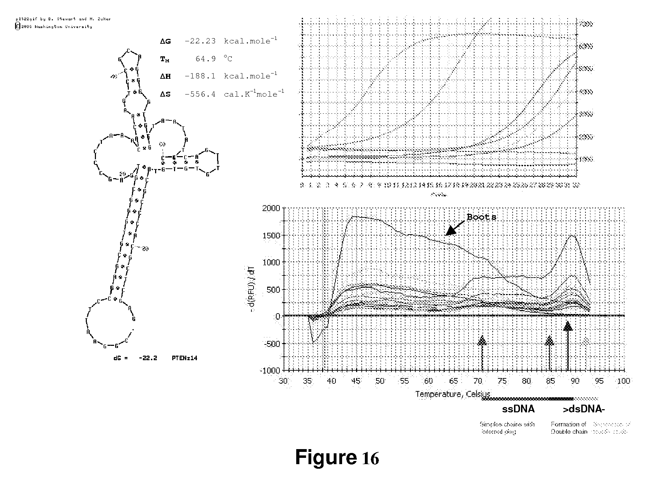

Figure 16 shows the theoretical structure predicted by a mathematical program (http://www.idtdna.com/scitools/Applications/mfold). According to the predictions, the structure of this oligo-antibody would be stable up to 64.5ºC. Amplifications of dilutions of this clone show how various amounts of the double helix are amplified by PCR (Figure 16, right). These PCR products are studied according to the number of double helixes and the temperature, showing that single strands are stable under 60ºC. According to the sequence, when they reach their temperature of melting (Tm), the head-tail bindings separate and the single strand forms with its pair the DNA double helix, increasing fluorescence as a result of higher incorporation of stain (figure 16, bottom right).

PCR products are stable between 65 to 85ºC, where the proportion of double-strand DNA rises up to 88ºC and finally they start separating again to remain single-stranded. The proportion of single strands seems stable enough to remain stable during incubation. Also, controls were performed with ten times more primers than usual (as the arrow shows). Chains are stable as predicted by the program, up to 65ºC, irrespective of the concentration (dark grey zone marked by arrows in the graph). Above 84ºC, chains are separated and join their reverse and complementary pair, forming a classic double strand (grey zone).

### BEHAVIOR OF CLONES IN NATIVE GELS

Chains produced by PCR are separated and run in a 7.5% polyacrylamide native gel. In these conditions, chains run according to the charge-to-mass ratio. The structure possibly also affects mobility, as two single strands and one double strand appear. In addition, a double strand links with itself to form a homodimer as the potential fourth chain (see diagram 4).

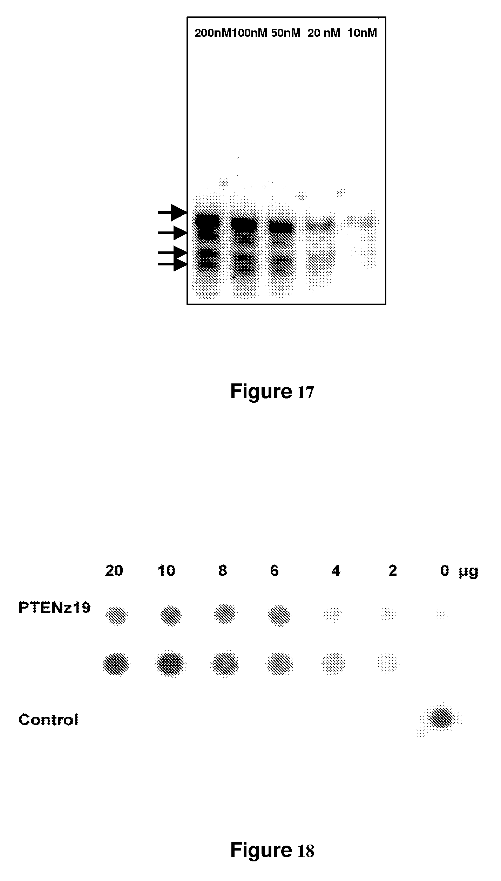

Figure 17 illustrates five different concentrations of PCR product marked with (32P)-dCTP. The thickest black arrow points at the potential double strand with maximum coupling efficiency.

### EXAMPLE 2.2

### REACTANTS OBTAINED RECOGNIZE THEIR TARGETS IN THE NANOMOLAR RANGE

### BINDING ASSAYS IN SOLUTION:

To carry out binding assays, pure recombinant proteins were used in filter binding assays. In these assays, the bind takes place in solution and then the reaction mixture is filtered through a nitrocellulose paper. This filter adsorbs peptides and proteins but not oligonucleotides, so that when radioactivity is retained it means that the ligand is bound to the protein. The following assay shows how a constant amount of radioactive ligand binds in decreasing protein concentrations. The filter is then visualized on a Phosphorimager Storm. Afterwards, dots are cut and radioactivity is measured using a Beckman scintillation counter using Cherenkov counting method.

Figure 18 visually illustrates the amount of radiation retained in the nitrocellulose filter. Mouse protein anp32e (cpd1) fused with GST and the oligo-antibody against the PTEN peptide were used as control, and 10 µg of recombinant cpd1 were used as positive control (bottom right). The amount of protein for GST is 10 µg, and 6 µg for the peptide.

### SCATCHARD CURVE OF LIGANDS FOR GST AND PTEN PROTEINS

A constant amount of protein is presented against decreasing concentrations of ligands synthesized with (32P)dCTP. The amount of bound radioactive is quantified with respect to the total unbound radioactive to determine the affinity constant of each ligand.

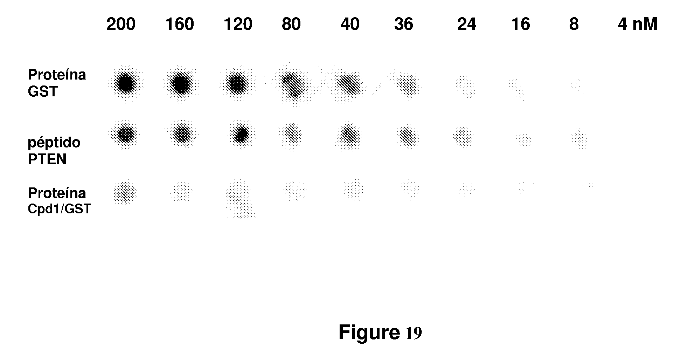

Figure 19 shows radioactivity retained by the proteins in the nitrocellulose filter. Each target (shown left) is presented against decreasing concentrations of ligands (shown on top).

### BINDING OF OLIGO-ANTIBODIES TO THE GST PROTEIN

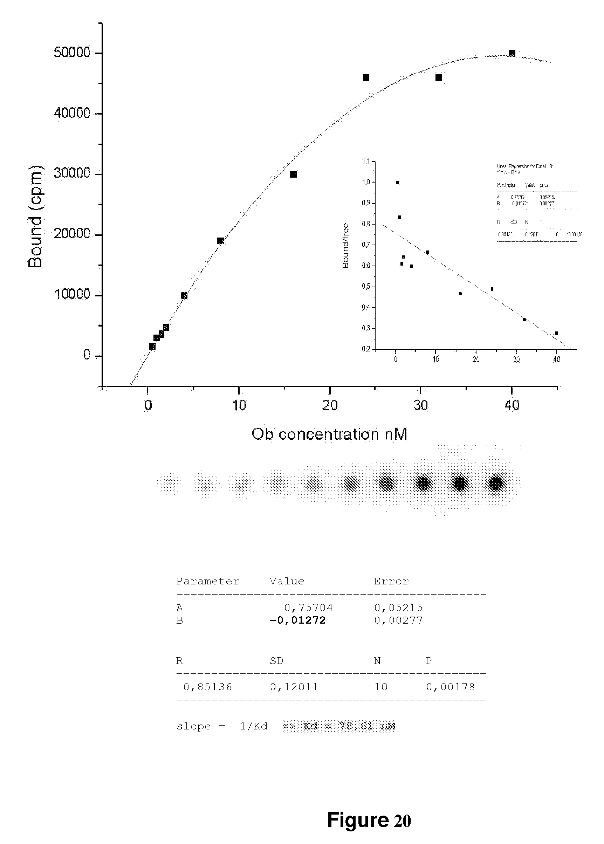

As shown in figure 20, the values are obtained by direct measurements of the radioactive contained in the collection point of the dish with holes in the nitrocellulose paper. The total number of operations used in each assay is measured beforehand, so the free ligand is the result of the subtraction of the total and the bound ligand. The resulting straight curve between the ligand concentration and the free portion over the bound portion defines the affinity parameters. The tangent defines the dissociation constant (Kd) and the affinity constant obtained is similar to that obtained for classical antibodies (10⁻⁷ M). B is the tangent and A is the origin of the straight curve. R is regression, SD is standard deviation, N is the number of specimens and P is the significance level.

### BINDING OF OLIGO-ANTIBODY TO THE PTEN PROTEIN

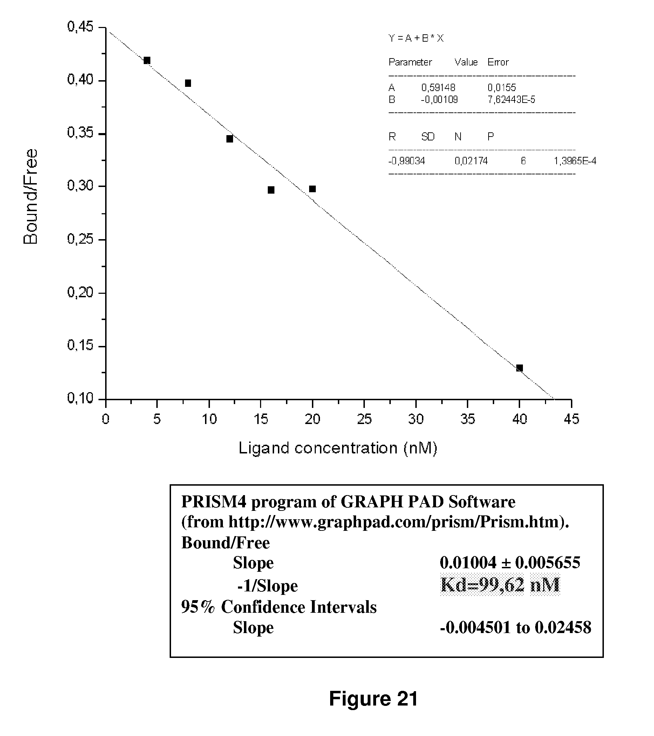

In figure 21, the slope obtained is B = -1/Kd **=> Kd = 94.4 nM**

When another program is used, the values obtained are comparable. The oligo-antibody too has an affinity close to 100 nM.

### THE SIMPLEX PROTOCOL PRODUCES LIGANDS WITH THE SAME AFFINITY USING DIFFERENT LIBRARY DESIGNS

The synthetic peptide against PTEN is used as target of a library containing a random region of 60 randomized nucleotides. In addition, the ends are not reverse or complementary, with a lower structure degree. One of the clones taken randomly was confirmed to be positive in the recognition of the recombinant protein and was used in the binding assay. This case shows that it is the selection rather than the structure the parameter limiting the affinity of the oligo-antibody.

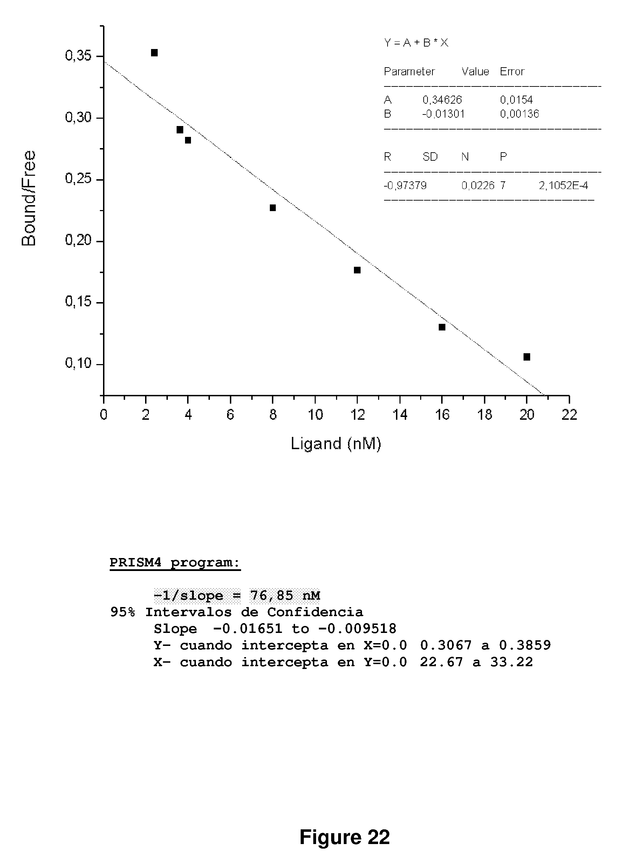

Figure 22 shows the slope obtained with the program Origin: -1/slope = 76.92 nM.

The values obtained correspond with those obtained by the other combinatorial library, showing the independence of the oligo-antibody structure type. Two different mathematical programs define the same affinity for this ligand, also in the nanomolar range.

### DETERMINATION OF AFFINITY USING A BIO-SENSOR: OLIGO-ANTIBODY AGAINST LYSOZYME

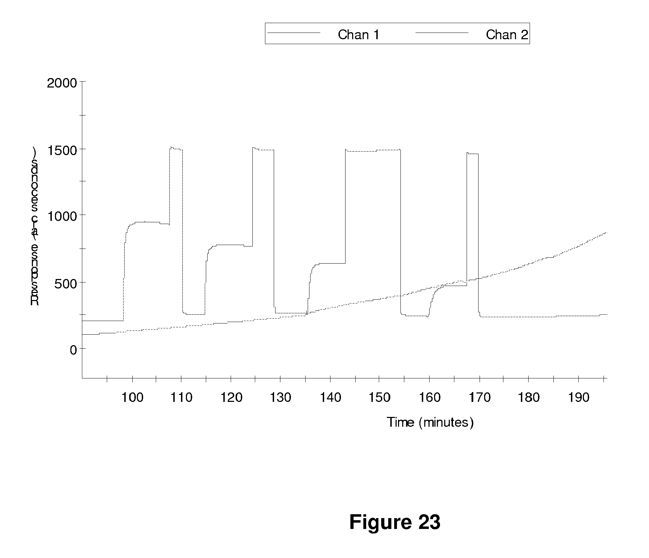

Using an affinity sensor (IAssays plus, Thermo labsystems) with the following conditions: Lysozyme bound to a carboxy-metil-cellulose matrix at high density and the liso3fw oligo-antibody were used in the measurement assay. Because the biosensor could not detect DNA, some innovations had to be made to detect it, the most important being the conjugation of the synthetic oligo-antibody with another molecule so as to detect it with the readers. Since the equipment is prepared for reading proteins, we used synthetic oligo-antibodies conjugated with biotin at the 5'-end and pre-incubated for 15 minutes with avidin. This binding allows us to assay the affinity of the ligand oligonucleotide as shown in figure 23.

The biosensor is a piece of equipment that records changes in light diffraction on a surface. The binding of a ligand produces diffraction changes (arc seconds, channel 1 in blue), which are proportional to the bound amount throughout time (on axis X, time in minutes). Bound ligands are washed with 1 M of NaCl and returned to the basic condition to start another measurement (seen as a dramatic rise to 1500 arc).

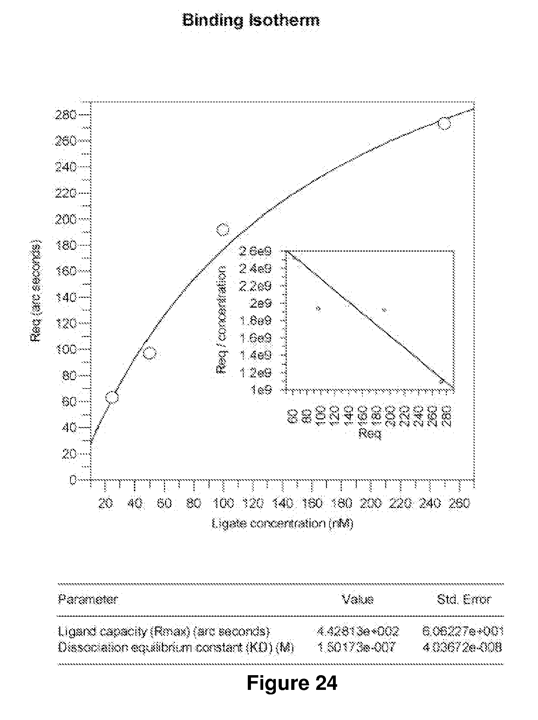

According to the values obtained in the above measurements, the constant R in equilibrium is obtained (Req), which depends on the ligand concentration. This way, the values can be extrapolated to a Scatchard curve as shown in Figure 24.

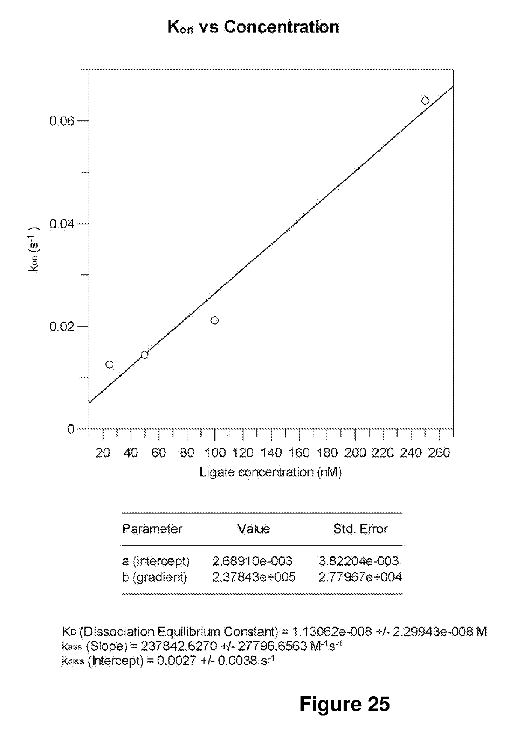

Extrapolating the values obtained, the dissociation constant can be defined as Kd = Koff/Kon. With this straight curve, both the dissociation and association constants can be estimated as shown in Figure 25.

The dissociation constant in equilibrium is estimated at 150 nM, again in the range obtained by other methods.

### EXAMPLE 2.3

### THE MIXTURE OF LIGANDS RECOGNIZES TWICE AS MANY SITES WITH THE SAME AFFINITY AS INDIVIDUAL CLONES

This example shows the affinity of each ligand for its target, comparing the affinity of the initial mixture. Three clones and one mixture of ligands without cloning are tested in filter retention assays in order to compare their detection capacity. The amount of ligands bound by the clone is half of that obtained with the mixture re-amplified with radioactive. In the example, the peptide corresponding with the first 12 amino-acids from the amino end of huntingtin. The amount of bound cpm gives a measure of the amount of targets recognized by a single clone against huntingtin, and we compare it with the re-amplified ligand mixture. Under 200nM, radioactivity in all clones drops.

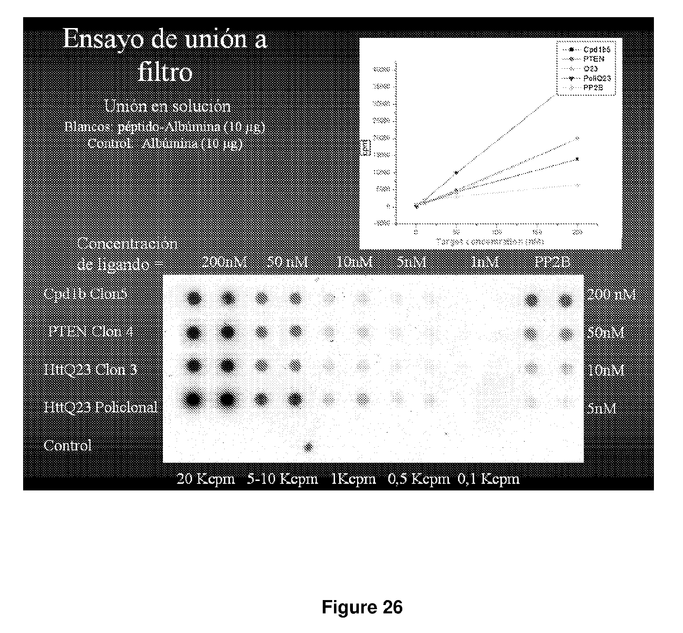

Figure 26 illustrates the amount of bound radioactive and the comparison of the mixture of ligands obtained. The twelve amino-acids bound covalently to bovine albumin are bound by twice as many counts as if only one ligand is used.

### EXAMPLE 2.4

### RETARDATION GELS: OLIGO-ANTIBODIES AGAINST PEPTIDES RECOGNIZE THE RECOMBINANT PTEN PROTEIN

Interactions between DNA and the proteins are analyzed through electrophoretic mobility shift assay. DNA is marked uniformly with ³²P phosphate using Termophilus aquaticus polymerase (1 nM DNA, 300,000 cpm). Chains are purified with columns of sefadex, opened by heating (95ºC during 5 minutes) and cooled down with 2 volumes of saline phosphate buffer. 5 µg of pure recombinant protein are added to the mixture and incubated 3 hours at room temperature. DNA-protein complexes are analyzed by electrophoresis in native polyacrilamide gels supplemented with 5% glycerol to stabilize constitutions. Gels are pre-run 15 minutes at 90 volts at 4ºC, and 25 µl specimen is seeded. The specimen is separated at 90 constant volts during 90 minutes; the gel is dried and exposed to the radioactivity reader screens.

Reactants: 30% acrylamide / 1% bisacrylamide (2.5ml) stock solution, TBE 5x (2ml), glycerol (0.5ml), enough H₂O for 10 ml, 1% of 10% ammonium persulphate p/v and 0.1% of TEMED (tetramethylethylenediamine). **TBE 5x** buffer: Tris-Base 0,5 M (60,5 gr/l) Boric acid 0,5 M (30gr/lt) EDTA 10 mM pH 8.

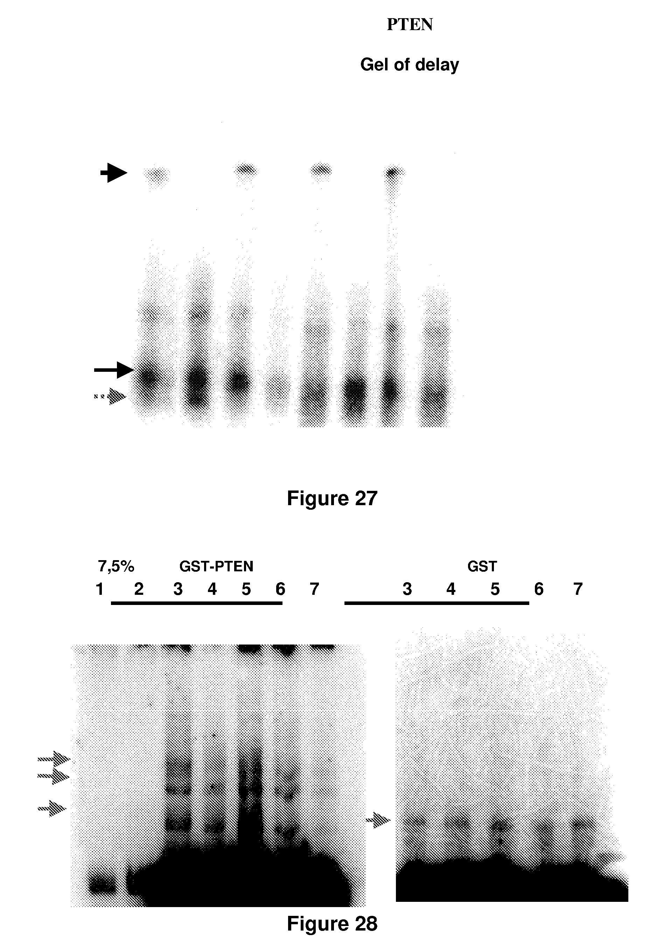

Figure 27 shows an image obtained by the Phospholmager Storm using (³²P) ssDNA as marked probe.

The oligo-antibody amplified in presence of (³²P)-dCTP by PCR and 10,000 cp are used in each assay. Chains are separated by heat and presented before 15 µl of pure PTEN/GST in Tris Buffer or Cpd1/GST as specificity control. Oligo-antibodies are incubated at 37ºC during 3 hours, and in order to discriminate between bound oligo-antibodies and unbound oligo-antibodies, the mixture is run in native gels. The mass-to-charge ratio of oligonucleotides makes them run with the front and when associated with their protein target their mobility is delayed.

### RETARDATION GELS: OLIGO-ANTIBODIES AGAINST GST RECOMBINANT PROTEIN

When the target used is a whole, denatured protein, the clones might nol recognize the protein in solution. Out of the clones obtained, four were selected which recognize the GST protein or the GST-PTEN chimeric protein.

Figure 28 shows Strands 1 and 2: PCR products are separated from the primers and radioactive dNTPs by a column of sefadex 200, and 50,000 cpm are run alone. The following lanes contain the mixture including 2 µg of pure GST-PTEN or GST protein. Rails 3 and 6 pertain to the **GSTp 9** clone; rail 4 pertains tc clone **GSTp13;** rail 5 to **GSTp7;** and lane 7 to clone **GSTp10.** The arrows show the delays suffered by PTEN and fragments of proteolysis (left) or by GST (center).

### RETARDATION GELS: OLIGO-ANTIBODIES AGAINST SPARC OSTEONECTINE/BMP40

SPARC (Secreted Protein, Acidic and Rich in Cysteine) is a protein whose structure is supported by a zone rich in cysteine and another zone rich in acid amino acids. In this example, recombinant SPARC is presented in its native form. Ligands obtained recognize SPARC in its native form with specificity. The affinity of oligo-antibodies is also in the range of 100 nM.

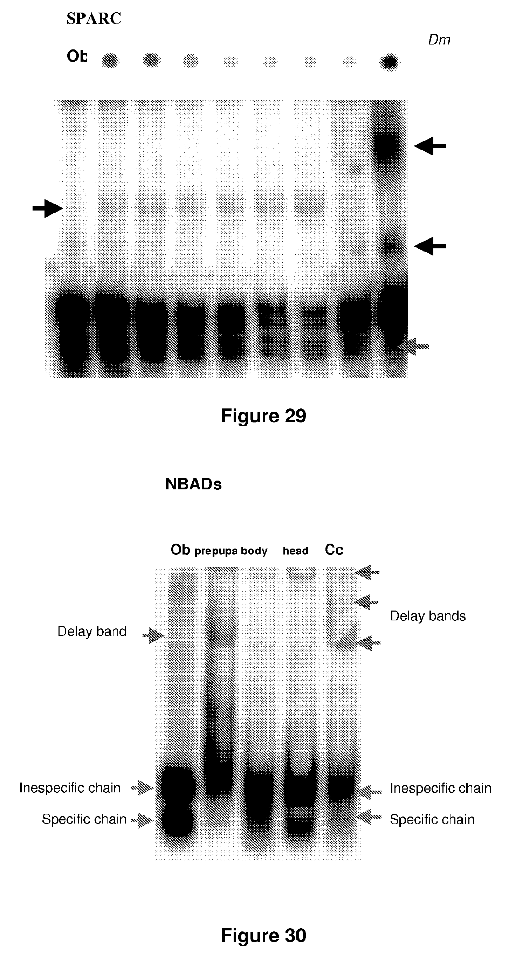

The oligo-antibody against SPARC (**Sparc 12**) recognizes the pure recombinant protein or the native protein in the protein mixture. The affinity of this oligoa-antibody is under 150 nM, as shown in the filter retention assay (Figure 29, top). In retardation gels, one band appeared in the presence of 1 µg of human recombinant protein as compared with the rail pertaining to the oligo-antibody alone (Ob). Ten times as much recombinant protein PTEN does not produce any retardation in the gel. When using a complex mixture of proteins from embryos of fly "Drosophila melanogaster" (Embryo Dm), only two bands appear. The lower front band clearly disappears in embryos (arrow).

### RETARDATION GELS: OLIGO-ANTIBODIES AGAINST RECOMBINANT PROTEIN N-β-ALANYL-DOPAMINE SYNTHETASE OR INSECT NBADS

This example shows how oligo-antibodies obtained against a protein enriched in bacteria can be used in biological assays to detect interactions in protein complexes. In retardation gels, proteins shift according to charge density (charge-to-mass ratio). This ratio is affected when a protein interacts with its pair, causing mobility delay in the gel.

NBAD synthetase plays several roles in insects' body cuticle and brain. Taking the mixture of proteins from Drosophila melanogaster pre-pupas, heads or bodies, we could observe that the NBADs protein is associated to various protein complexes, causing several delays. In ceratitis capitata (Cc), NBADs is associated with immune response in the cuticle. This different function is accompanied by a new complex with the oligo-antibody (NBADs-9). The protein mixture only binds one of the two chains, showing that specificity is due to the sequence (chain-specific). Figure 30.

### EXAMPLE 3.1

### PRODUCTS OF THE SIMPLEX PROCEDURE: CHARACTERIZATION OF LIGANDS IN DETECTION ASSAYS IN MEMBRANE

### EACH DNA LIGAND IS CAPABLE OF RECOGNIZING THE TARGET INDIVIDUALLLY AND SPECIFICALLY

Ligands eluted from the membrane are taken to double chain using Taq polymerase and bound in bacterial plasmid. Bacteria transformed with the vector are isolated, and each clone obtained is analyzed. The plasmid is purified in each of the clones to analyze their sequence, and the inserted oligo-antibody is amplified by PCR using primers marked with biotin.

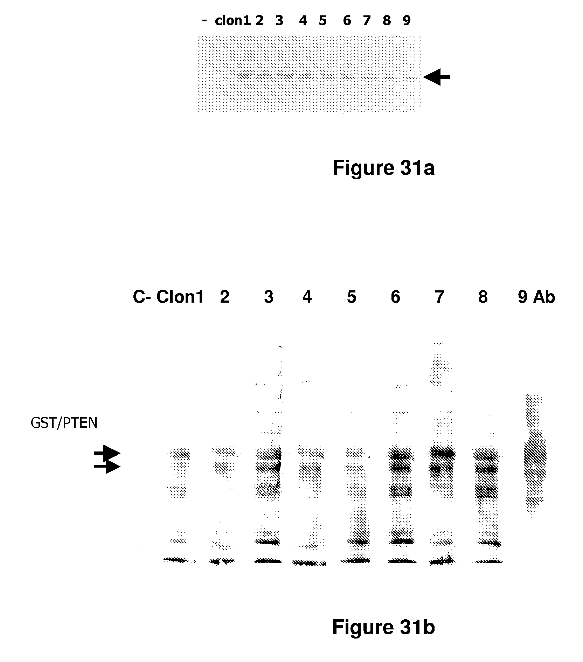

Ligands obtained by PCR are analyzed in 3% agarose gels and visualized with ethidium bromide. All colonies analyzed had insert of 87 bases amplified by polymerase (black arrow). Colonies are numbered 1 to 9 on the upper edge (Figure 31 a).

Amplified products are used in detection assays in membrane with a mixture of proteins containing recombinant protein PTEN. 70% of clones recognized PTEN bound to Glutation-S-Transferase (PTEN-GST), and all retain their specificity in the recognition of the target peptide.

### RECOGNITION OF PURE RECOMBINANT PROTEIN FOR EACH LIGAND IN COMPLEX MIXTURES

The oligo-antibody from each clone is capable of recognizing its target even in complex protein mixtures. This example shows how each clone is capable of recognizing PTEN fused with GST and expressed in Escherichia coli cultures. Proteins obtained from these cultures are separated by molecular size through electrophoresis in polyacrilamide gels in presence of SDS and transferred to a nitrocellulose membrane. Membranes are incubated with oligo-antibodies and developed with the detection system using alkaline phosphatase.

Bacterial lysate without plasmid does not produce any band resulting from the non-specific bind with alkaline streptavidin-phosphatase used for recognizing biotin in the oligo-antibody (C-). PCR products of each marked clone (200nM) are incubated with the proteins of bacteria transferred to membranes overnight. These are washed three times with PBS and recognized with streptavidin and alkaline phosphatase using NBT/BCIP chromogens. The arrows point at the position of the recombinant protein and a processing product of smaller size and lesser mobility (thin arrow). The recombinant protein is recognized as well for the commercial antibody used as positive control in strand 9 (9Ab). (Figure 31 b).

### EXAMPLE 3.2

### RECOGNITION OF TARGETS IN WESTERN BLOT ASSAYS

2 µl of library (200 nM) is amplified by 15 cycles. After the selection against peptides of PTEN and Cpd1, SIMPLEX products are amplified with 15 PCR cycles in presence of (³²P)-dCTP. In this way, open chains are presented to mixtures of proteins from cerebellum homogenates separated from polyacrilamide by electrophoresis. The membrane is blocked with bovine serum albumin, which is also used to compete non-specific interactions during incubation. After 6 hours of incubation with the oligo-antibody, membranes are washed out of the unbound reactant and the radiation is detected by radioactive-sensitive screens after 45 minutes of exposure. The reading of the screens in the Phosphoimager Storm shows the mark pattern of detected bands, which corresponds with those described and expected for each target.

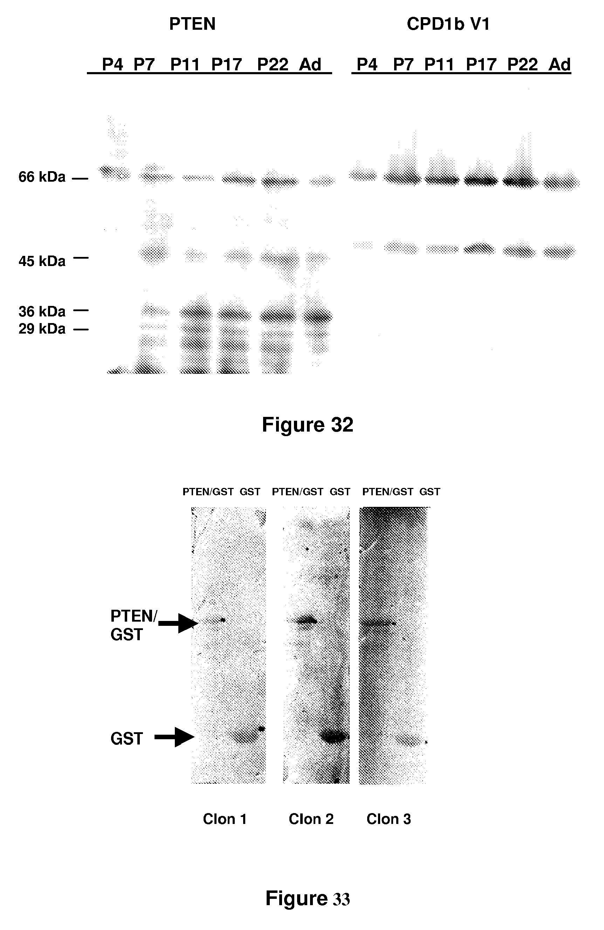

The image in figure 32 shows the mark distribution detected by each mixture of oligo-antibodies both in the molecular size (shown in kDa left) and in the intensity present at each post-natal age (as post-natal days indicated on top, P#). In addition, the different development periods of the murine cerebellum and the variation in the intensity of the expression are compared. This result shows that the mixture of selected oligo-antibodies is capable of recognizing specific targets in various complex mixtures.

### EXAMPLE 3.3

### GLUTATION-S-TRANSFERASE OR GST: RECOGNITION OF THE NAÏVE PROTEIN AND RECOMBINANT PROTEIN BY THE OLIGONUCLEOTIDE LIGANDS

Another oligo-antibody was Developer using denatured GST protein in a nitrocellulose as selection receptor. From this selection, three clones were determined which were capable of recognizing the protein both in its native form and the recombinant protein with PTEN.

Proteins GST and GST-PTEN were expressed in bacteria and purified in affinity columns with glutation. These proteins were denatured, separated in SDS-PAGE and transferred to nitrocellulose membrane. Membranes blocked with bovine serum albumin were incubated during three hours with oligo-antibodies marked with biotin with a concentration of 200 nM. The bound oligo-antibody was detected with alkaline phosphatase bound to streptavidine (Figure 33).

### EXAMPLE 3.4

### OLIGO-ANTIBODIES RECOGNIZE AMINOACID DIFFERENCES IN A 14-RESIDUE PEPTIDE

ANP32 family members can be differentiated by changes in their protein sequence. The E member, i.e. anp32e, differs from the others in that it has tyrosine instead of histamine. In order to determine the specificity of the reactants obtained by the SIMPLEX method, two synthetic peptides were used as target receptors and the selection was performed by subtracting the libraries.
Synthetic Peptides
CPNLT**Y**LNLSGNKI (Anp32e/Cpd1/Lanp-I) and
CPNLT**H**LNLSGNKI (Anp32a/PHAPI/Lanp and Anp32b/April/ss29 proteins).

Three different clones were analyzed in "western blot" assays using single-strand DNA probes marked with ³²P-dCTP and murine cerebellum protein separated in SDS-PAGE gels 10%. Three different mouse ages were used, as it is known that there are differences in the protein expression during development (P7, P17, Ad). After incubating the probe overnight, the rinsed membrane is exposed to radioactive-sensitive screens for half an hour and finally developed using a Phosphoimager Storm.

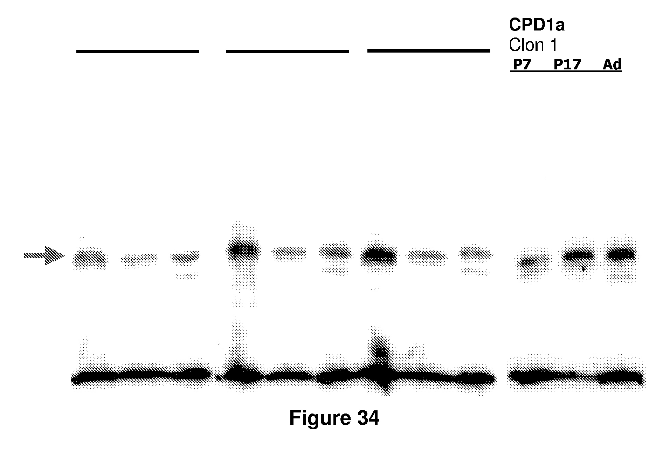

Figure 34 shows the image obtained, where it is observed that the three clones against the PHAPI peptide are repeated in the double-stranded 32 kDa pattern, with a lower expression at day 17 postnatal (P17). For the same specimens, Cpd1 a detects Anp32e that reaches its maximum expression at this age and remains high in the adult (AD). (Changing tyrosine amonoacid for hystamine, T => H).

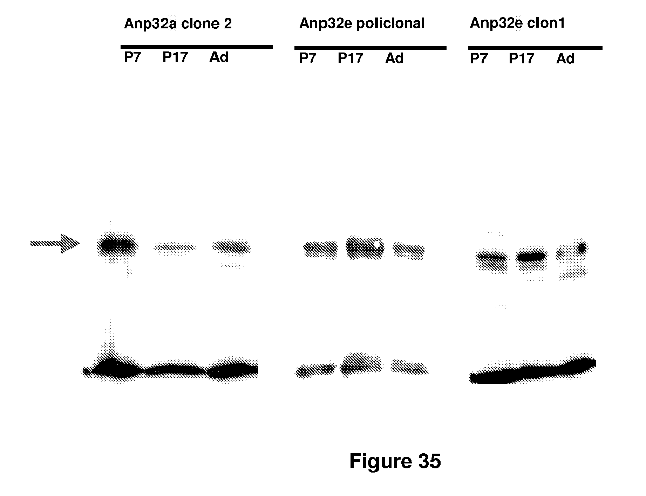

Figure 35 compares the detection of the E member of the Anp32 family relative to the other members which contain histamine in the selected peptide (members a, b , c, d, respectively). When the results are compared, the differences in the intensity of the protein 32-kDa remain both in the olig-antibody mixture (polyclonal) and in the specific clon. All the oligo-antibodies showed the presence of mark on the front of the run; this may be attributed to the degradation of the native protein.

### EXAMPLE 3.4

### DIFFERENTIAL RECOGNITION OF PROTEIN ANP32e ISOFORMS.

Anp32e or Cpd1 has four different molecular weight isoforms. When the ligands obtained against the Anp32e peptide were analyzed, one of the clones only recognizes the 66 kDa isoform in solution and the other isoforms, whenever the proteins are denaturalized in western blot assays. The figure shows the result of Anp32e protein purification by using oligo-antibodies with biotin and magnetic micro particles with binding streptavidin. Purified protein is run with gels and transferred to nitrocellulose to be analyzed with antibodies against another peptide corresponding to Anp32e. This assay shows the 66 kDa isoform as the only one detected by the antibody. In western blot assays, the antibody recognizes at least three isoforms in proteins deriving from an adult mice cerebellum (2nd Strand, adult: AD). The oligo-antibody recognizes three bands at the studied ages (arrows) and the thick arrow shows the position of the 66 kDa band. Protein purification is made under native conditions, preserving interaction with other proteins. In this way, proteins linked both to Anp32e (Cpd1) and to PTEN are co-purified. Purification products (Ipp) are run with polyacrylamide gels and undergo a silver staining process.

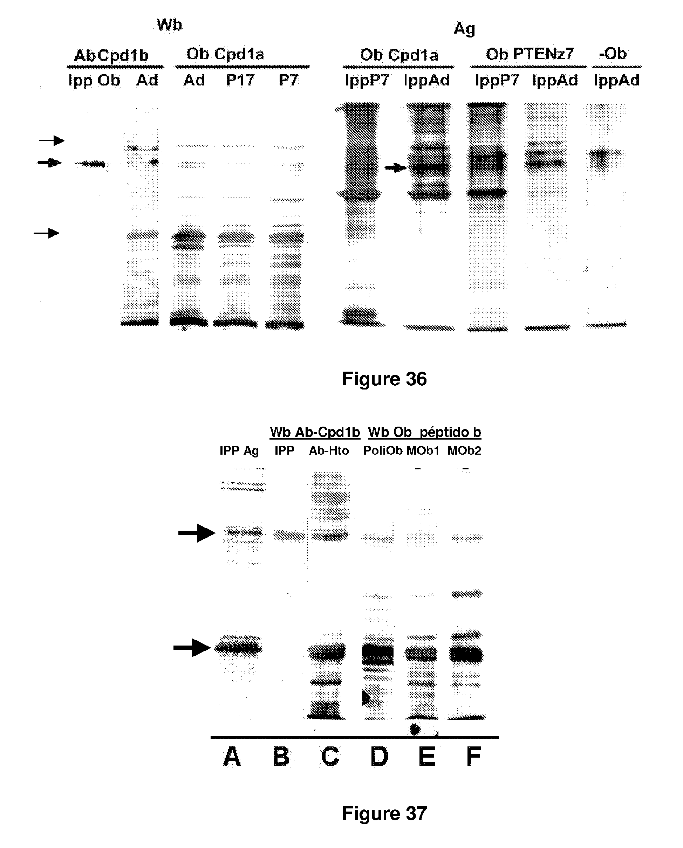

Figure 36 shows the identification of protein 66 kDa as an isoform recognized by the oligo-antibody against the peptide "a" (CPNLTHLNLSGNKIK) and recognized by the antibody of another peptide "b" (VLDNCLCVNGEIE) used as antigen. Protein homogenate from a murine cerebellum was used for western blot assays or to purify proteins recognized by the oligo-antibody and its related proteins. Proteins were run with 12.5% polyacrylamide gels and transferred to nitrocellulose sheets (WB) or stained with silver (Ag). Western blot assays were made using rabbit antibody, 1 in 400 diluted, and oligo-antibodies (Cpd1 and PTEN) were incubated at a concentration of 200 nM.

### EXAMPLE 3.5

### OLIGO-ANTIBODIES AGAINST 74 kDa ISOFORM OF ANP32E/CPD1.

Ligands obtained with the SIMPLEX method against the "b" peptide of Anp32e (VLDNCLCVNGEIE) are used as a mixture in solution to purify the protein to be subsequently identified as rabbit antibodies. Figure 37 shows the purification by means of capturing magnetic particles shows two majority bands in polyacrylamide gels stained in silver (IPP Ag). The upper one is of 74 kDa and is identified by the antibody (IPP), matching its mobility to that of the protein detected in cerebellum homogenate.

In western blot assays using oligo-antibodies, 74 kDa bands are detected, an extra one of 49 kDa and a double majority band of 32/34 kDa. Clones can recognize a peptide as a point (at the base of strand E).

Proteins are the sub-cellular fraction of neuronal connections (synapses) deriving from 17 postnatal day murine cerebellum. Protein staining was performed with "Coomasie blue" and shows the two majority bands (arrows in A, figure 37). The identity of the upper band was performed with the antibody made up with the conjugated peptide of bovine albumin and inoculated on rabbits. The 74 kDa band was marked at purification (B) and is also recognized in homogenates (C). The mixture (D) and the clones (E and F) are capable of recognizing the same band pattern as a rabbit's antibody. The lower band resulting from the purification was identified by means of the mass pattern analysis from fragments triggered by digestion with trypsin.

### EXAMPLE 3.6

### CONFIRMATION OF THE IDENTITY OF HUNTINGTIN PROTEIN RECOGNIZED BY THE OLIGO-ANTIBODY HTT5.

The purpose of this assay is to identify the target known by those species capable of recognizing the peptide and not the recombinant huntingtin protein. The assay is performed by purifying huntingtin from the extract of three brain regions from the mouse with the oligo-antibody Htt5 and by identifying it with commercial antibodies.

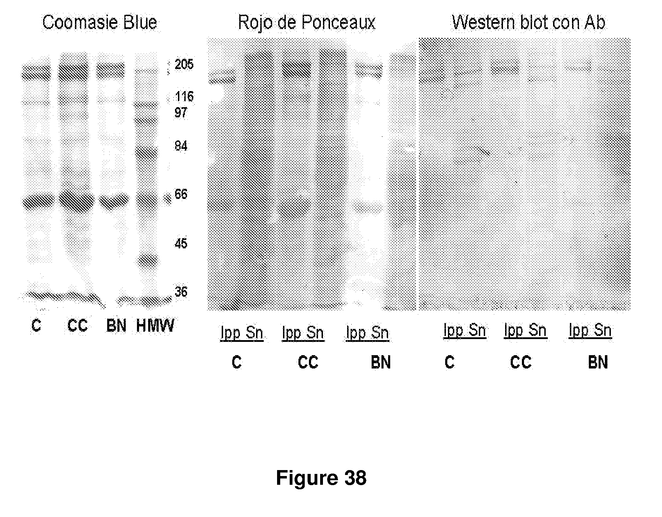

Figure 38 shows purified proteins by means of oligo-antibodies and separated in polyacrylamide gels. They are stained with "Coomassie blue" in order to determine the number of proteins present and a twin gel is transferred to the nitrocellulose membrane and temporarily stained with Ponceaux red. The different brain regions (C: cerebellum; CC: cerebral cortex; BN: basal nuclei) are separated in the beaker with oligo-antibodies (Ipp) and the homogenate supernatant is used for purification (Sn). The membrane incubated with the antibody against the Huntingtin protein (Santa Cruz biotechnology) is subsequently developed with a secondary antibody coupled to alkaline phosphatase. The antibody recognizes the two high molecular weight anybodies (indicated with arrows) and not the 66 kDa protein, which identity has not been yet clarified. Notably, the Htt5 oligo-antibody is capable of distinguishing a native protein from a recombinant protein.

### EXAMPLE 3.7

### SIMPLEX LIBRARIES MAY BE APPLIED TO A PROTEIN ENRICHED IN COMPLEX SPECIMENS: FLY RECOMBINANT N-β-ALANIL-DOPAMINE SYNTHETASE (NBADS).

In this example, evidence is provided as to how it is possible to use a protein enriched mixture, chosen as target. This is the case of proteins introduced and expressed in bacteria. Protein expressed in bacteria are run in SDS-PAGE and separated by molecular weight, transferred to membranes and stained with ponceaux red. The majority band is cut and used to obtained ligands with the SIMPLEX protocol. Contrary to other incubations, bacteria protein that does not express the enzyme used in order to block ligands obtained against contaminated proteins (A). Chosen ligands were tested as mixture or as a unique chain (cloned) against protein obtained from bacteria with a different induction grade (IPTG). These clones recognize the whole protein purified by affinity columns or in homogenates (panel C). The results obtained show that it is not necessary to have a pure sample in order to develop the method and obtain specific ligands.

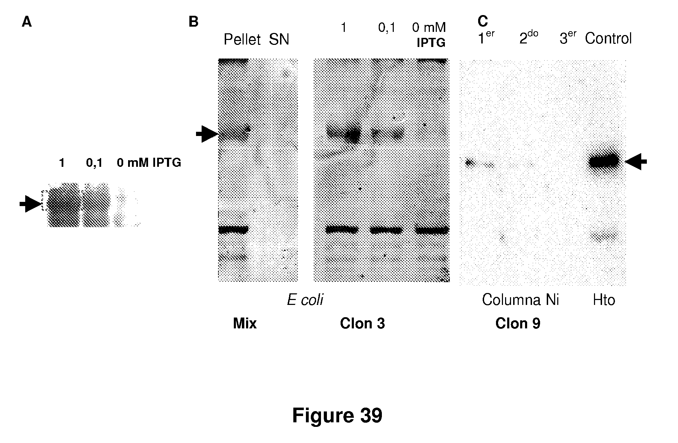

Figure 39 shows the protein pattern separated by electrophoresis in a 7.5% denaturalized SDS-PAGE gel and transferred to a nitrocellulose membrane. Proteins are temporarily stained using Ponceaux red and IPTG induced protein appears on the first panel (**A**). The red dotted line shows the cut membrane that will be used as target before the extended library. The second panel (**B**) shows the western blot deriving from the induction of the NBADs N-terminal fragment induction, before the total ligands mixture obtained in the selection. Purified plasmid from each clone is used as template in chain polymer reactions (template appears at the bottom of the figure). The mixture reveals the incomplete protein on the insoluble inclusion bodies (Arrow) and is not detected with soluble proteins. The same band is detected by clone 3 in amounts consistent with the induction pattern (IPTG, mM concentration range). The whole NBADs protein is soluble and includes a Polyhistidine tail enabling it to be purified by affinity to Nickel (Ni) in one column. The elutions are separated by electrophoresis and transferred to a nitrocellulose membrane. The ninth clone plasmid is used as template in a PCR that contains ³²P-dCTP and products separated to simple chain to be used as probe with the complete NBADs after purification. The third panel (C) shows the footprint left by radioactivity bound to the pure protein or in soluble protein homogenate (Hto, arrow). The numbers indicate whether this is the first, second or third elusion of the Nickel column, while the arrow indicates the expected molecular weight for the whole NBADs protein.

### EXAMPLE 3.8

### TARGET CONSTITUTION IN THE PRESENTATION IS DIFFERENTIATED BY PRODUCTS FROM THE SIMPLEX PROCESS. SPARC PROTEIN.

SPARC protein is used as target in its native constitution supported on a nitrocellulose membrane. The oligo-antibody against SPARC does not recognize the denaturalized protein in polyacrylamide gels and whether it is the native form in complex protein mixtures or a pure recombinant protein.

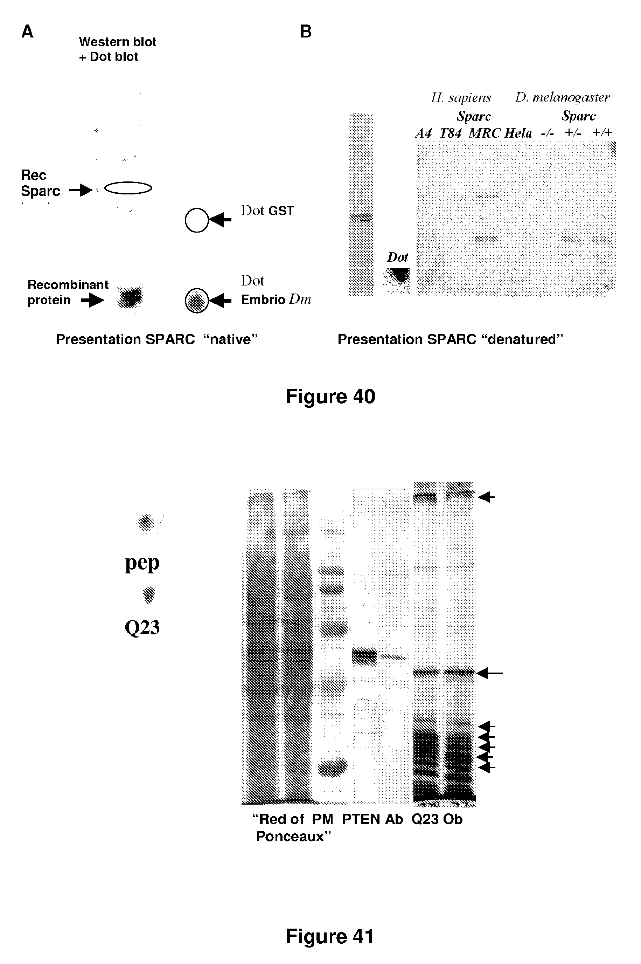

Figure 40, panel **A** shows that the oligo-antibody against SPARC is presented to 5 µg of pure protein (Rec-SPARC), opened with B-mercaptoethanol and ran on polyacrylamide gels with SDS detergent. The nitrocellulose transferred protein is not recognized by the oligo-antibody under such denaturalizing conditions. The protein is recognized whenever it is presented in a native form in a "dot blot" either pure or in mixtures.

### EXAMPLE 3.9

### IT IS POSSIBLE TO SELECT SPECIAL TARGET CONSTITUTIONS: HUNTINGTIN AS PEPTIDE AND AS PART OF A CHIMERIC PROTEIN.

Previous results showed how oligo-antibodies (were) obtained against the first 12 terminal Huntingtin amino acids. The latter discriminate this sequence when the Glutathione-Sulpho-Transferase fusion protein internally meets with the first 171 amino acids (GST-Htt171). A new peptide selection achieves a mixture capable of recognizing both the peptide and the recombinant protein. In neuronal cultures, such mixture (Q23) is capable of recognizing the whole huntingtin protein (350 kDa, on the upper limit of the run) and several fragments smaller than 50 kDa (arrows).

Figure 41 shows the detection of proteins and peptides from different origin. The mixture recognizes both the peptide (pep) as well as the recombinant protein (Q23). The same amount of proteins were separated in a 10% SDS PAGE gel and transferred to membranes. Staining with Ponceaux red shows the protein charge and the molecular weight (MW) standards. Two lanes were used with the PTEN antibody (PTEN Ab), the remaining two are incubated with Q23 (Q23 Ob) oligo-antibody amplified with primers containing biotin and developed with streptavidin -alkalin phospahatase. Molecular weight standards correspond to 205 kDa, 116 kDa, 98 kDa, 66 kDa, 45 kDa and 29 kDa.

### EXAMPLE 3.10

### OLIGO-ANTIBODIES CAN BE INCORPORATED IN BIGGER MOLECULES PRESERVING THEIR SPECIFIC DETECTION CAPACITY.

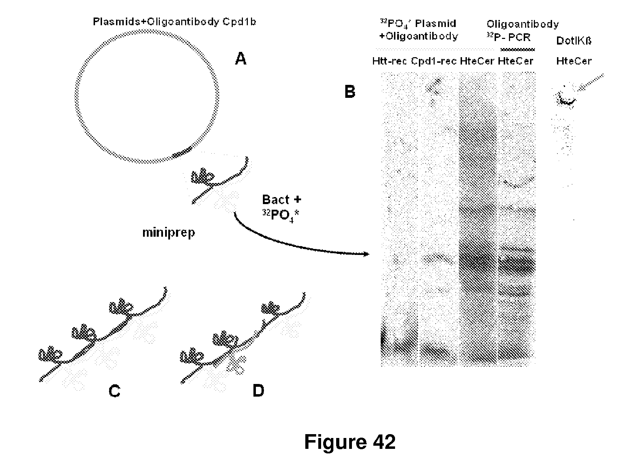

Oligo-antibodies are inserted within expression vectors, made up of a double stranded-DNA molecule which folds up and makes up a ring called plasmid (A, figure 42). The plasmid is incorporated to bacteria developing resistance to antibiotics and such bacteria are also developed in presence of the ³²PO₄. radioactive. The oligo-antibody recognizes the whole purified, denaturalized plasmid as a recombinant protein, that was used as a probe in western blot assays (panel B). The recombinant GST-Htt171 protein does not provoke any band, with the exception of the front of the run (Htt-rec). The Cpd1 oligo-antibody is capable of recognizing a band (Cpd1 rec) when it is incubated in presence of a protein mixture of E. coli that contains Cpd1 protein (sic). When it is incubated before a mixture of proteins from the cerebellum (HteCer) plasmids achieve the same pattern as with the clone without the plasmid (lane in red, Oligo-antibody with ³²P incorporated during PCR). The plasmid containing an oligo-antibody against IK- β only recognizes the protein in the "dot blot" assay (dotlKB, grey arrow), showing that bands obtained in the cerebellum homogenate (HteCer) correspond to the oligo-antibody sequence against Cpd1. Such property in the design of aptamers allows for the increase of the quantity of a ligand (C) or for the production of complex oligo-antibodies mixtures (D).

### EXAMPLE 3.11

### THE DNA PROBE IS CAPABLE OF RECOGNIZING THE PROTEIN ON HISTOLOGICAL SECTIONS

### PROTOCOL for HISTOCHEMISTRY with OLIGO-ANTIBODIES

**1-** Incubate sections at 58°C for 30 min.
**2**- Soak sections following the sequence below:

| | |
|---|---|
| xylol | 20 min. |
| Ethanol 100% | 10 min. |
| Ethanol 70% | 10 min. |
| Ethanol 50% | 10 min. |

**3-** *Rinse with distilled water and carry out an antigen recovery process or "antigen retrieval". To such end, heat the citrate buffer with 0.05% Tween-20, at 90-95°C. Soak the already de-paraffined sections, let them rest for 20 min. at 95-100 °C and then remove them and let them cool for 20 min. at room temperature. Rinse twice with PBS. (* optional)
**4-** *Treatment with DNAse. Sections are covered with a buffer of DNAse RQ1 (10x: Tris CIH 400 mM pH 8,0; 100 mM Mg SO₄ 10 mM ClCa. 1 ml of MIX uses 40 µl of DNAse) and are incubated for 30 min at 37ºC. The incubation stops at 65 ºC for 10 min. in EDTA 5 mM. Sections are rinsed twice with PBS. (* optional)
**5**- Block non specific reactions by incubating 30 min. with 2%PBS/BSA
(2g bovine albumin /PBS, vf=100ml) 100 µl each section (aprox.)
**6**- Withdraw the blocking solution and incubate ON, at 4°C, with 100µl of:
- 1^{st} antibody incubation (primary): 1/100 primary antibody dilution in PBS BSA 2%. (5 µl /1000 µl-PBS 2% BSA).
**ó**
- 1^{st} Oligo-antibodies incubation. 1/125 Dilution OB PTEN mixture 200 nM each one, in 2%PBS BSA. (Dilute 8 µl of mixture of OB -2µl each one- in 100 µl PBS). Heat at 95°C 2-3 min. to ensure DNA solubility. Bring to volume by pouring 100 µl at 95º C onto an eppendorff at 4º C with 900 µl of PBS-BSA at 4 ºC. (IMMEDIATELY)
**7**- Remove the solutions and rinse once for 10 min. with PBS. Incubate with

| | |
|---|---|
| 1.5% Hydrogen peroxide in PBS | 10 min. |
| PBS | 10 min. |

Incubate 1 hour with 50/100 µl of :
- Incubation of anti-murine antibody marked with biotin (Sigma): 1/200 antibody dilution in 2%PBS/BSA, 180 minutes. Three 10-minute wash-outs with PBS.
- Detection: ABC Vector Labs. Staining kit with peroxidase.
   1 drop of A + another of B at least twenty minutes before incubation in a 2.5 ml volume. Sections are covered with two drops of mixture (100 µl aprox.) and incubation is performed for 30 minutes or more.
**8**- Remove the solutions and perform 3 10-minute wash-outs with PBS.
**9-** Incubate with developing solution until color becomes evident. Contrast staining with hematoxylin. (30 sec. in hematoxylin and washout in tap H₂O where color sets from violet to blue).
**10**- Once sections have been developed start with the dehydration sequence:

| | |
|---|---|
| PBS | 2 min |
| Ethanol 50% | 10 min |
| Ethanol 70% | 10 min |
| Ethanol 100% | 10 min |
| Xylol | 15 min |

Place a drop of Canada balsam on each section and place over a slide. Dry completely before putting the slide away (ON-24hs).

**Development solution for peroxidase**

| | |
|---|---|
| DAB 10 mg/ml | 50µl |
| PBS | 950µl |
| Hydrogen peroxide | 20µl |

Prepare at time of use.

**Citrate Buffer**

| Solution **A** | | Solution **B** | |
|---|---|---|---|
| Citric acid | 4,2g | Citrate sodium | 14,7g |
| Distilled water up to 200 ml | | Distilled water up to 500ml | |

| Working solution | | | |
|---|---|---|---|
| Solution A | 18 ml | | |
| Solution B | 82 ml | | |

Adjust pH to 6 and incorporate 0,5 ml of Tween-20. Bring it to 1 It. with H₂Od
**STOP solution of DNAse RQ1:**
Buffer EDTA 5 mM
Stock solution of EDTA 500 mM 1/100 diluted

### HISTOCHEMICAL RESULTS USING OLIGO-ANTIBODIES: MURINE BRAIN SECTIONS

Tissue sections come from mice having different postnatal ages, fixed with acid alcohols and subsequently paraffin-embedded. Paraffin is removed with Xylol; the sections are rehydrated with an alcohol gradient and non-specific binding sites are blocked with bovine albumin. After the blockade, sections are incubated for three hours in presence of the oligo-antibody at 200 nM or higher concentrations.

This example was performed to show the detection capacity of the oligo-antibody with PTEN, ANP32e/Cpd1 and SPARC protein in histological brain sections during postnatal development. Oligo-antibodies come from the initial PCR mixture using primers containing biotin which will be detected with streptavidin bound to peroxidase and developed with peroxide and precipitant chromogens (3-3, di-amino-benzidine tetra hydrochloride, which oxidation provokes a brown insoluble precipitate). 5 µm sections were incubated three hours at room temperature with a 200 nM oligo-antibody concentration. Age selection correspond to two morphogenic periods (P7 and 17) and adult. Those affected by weaver mutation are also compared.

### HISTOCHEMICAL RESULTS IN MURINE BRAINS

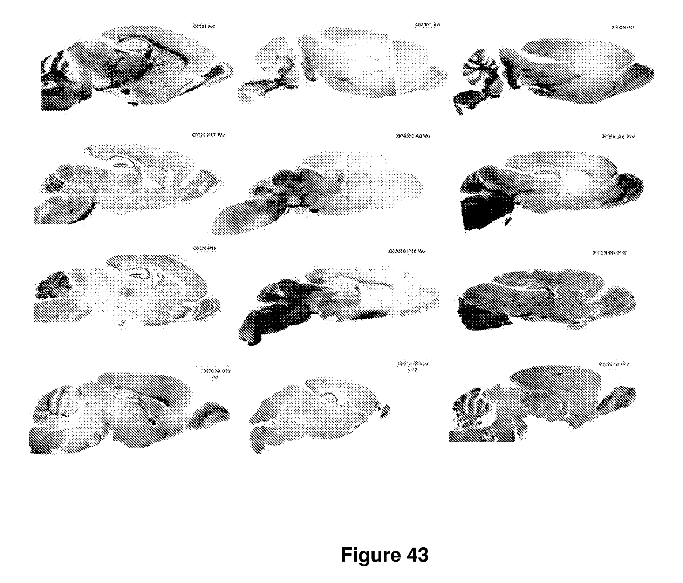

Figure 43 shows different parasagital sections from murine brain. Each section has on its upper part a mark indicating the identified protein, the mouse age or whenever it is a mutant genotype (mutant cerebellum weaver: Wv). The image is magnified twice and peroxidase is seen as black in the section, without contrast staining. Note that the location pattern developed by the staining process differs from the selected target, the age or genotype.

### HISTOCHEMICAL RESULTS USING ANTIBODIES AND OLIGO-ANTIBODIES AGAINST PTEN.

The cytoarchitecture of the cerebellum is made up of three clearly defined layers, which size varies during postnatal development. Grain cell productive layer is temporary and appears at early stages in the external layer of the cerebellum cortex and is therefore known as the external grain layer (EGL). Below, is a layer with scarce number of cells known as the molecular layer (MI). This is a layer where the Purkinje cell dendrites are found, having cell bodies aligned in a layer bordering the internal part of the molecular layer. The inside of the cerebellum cortex is made up of a internal grain layer (IGI). Oligo-antibodies directed against the recombinant PTEN protein showed a cytoplasmatic and nuclear location pattern.

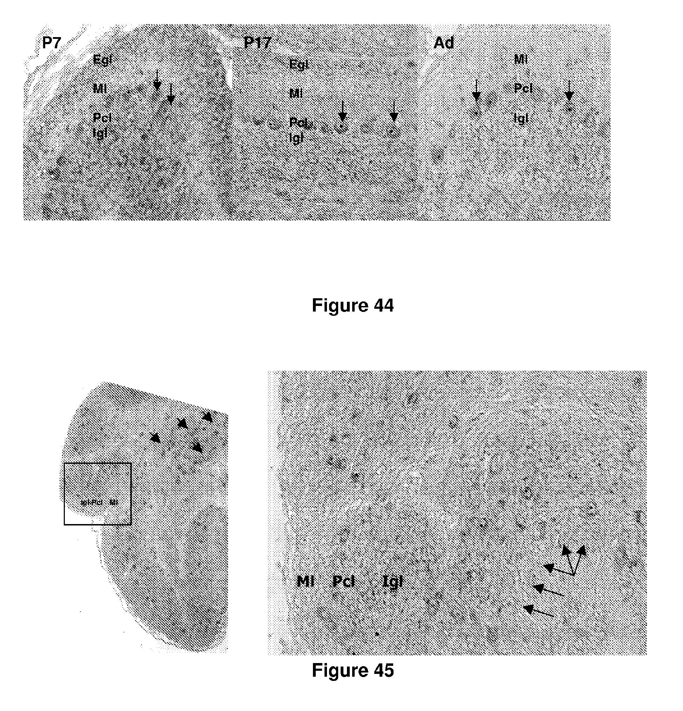

Figure 44 shows microscopes from the preoxidase staining performed at the three ages, magnified 200 times. Postnatal 7 or P7 is the maximum migration age of cells that make up the EGI which slims down at P17 and disappears in the adult. The IGL gains weight due to the contribution of cells migrating from the external layer, and the Purkinje cells make up the Pcl. These cells constitute the best example to show nuclear and cytoplasmatic staining which is also seen in the remaining smaller sized cells.

"Staggerer" is a genetic mutation (RORa) affecting the molecular signaling means in the cerebellum of a rat. When we use the oligo-antibody in these mutants we can see the absence of PTEN exclusively on the cytoplasm of Purkinje neurons. These results are consistent with what is expected from this signaling pattern in this genotype, reasserting the capacity of detection of oligo-antibodies mixture against PTEN.

Figure 45 shows the test magnified four times on staining made with the oligo-antibody mixture against PTEN in "staggerer" mutant mice at P17. The marking shows the absence of PTEN in cytoplasm of Purkinje cells and not in cerebellum nuclei (arrows) macro neurons. When magnified 40 times, this pattern shows that the cytoplasmatic staining is clearly absent from these neurons (area marked with arrows). Other neurons, making up the IGI, keep such marking, as observed at both magnifications.

### OLIGO-ANTIBODIES HISTOCHEMISTRY AGAINST PTEN USING ENDOMETRIUM.

PTEN protein is a remarkable tool for the early diagnoses of endometrium cancer. PTEN loss precedes the malignancy of endometrium cancer tumors in hyperplasia. We use this human genetic model to verify the specificity of the oligo-antibody mixture in this model where the PTEN loss inexorably occurs in 100% of adenocarcinoma. Tissue chosen is an abnormal endometrium hyperplasia where glands coexist in the presence of PTEN and without it, and may be considered as internal control/control.

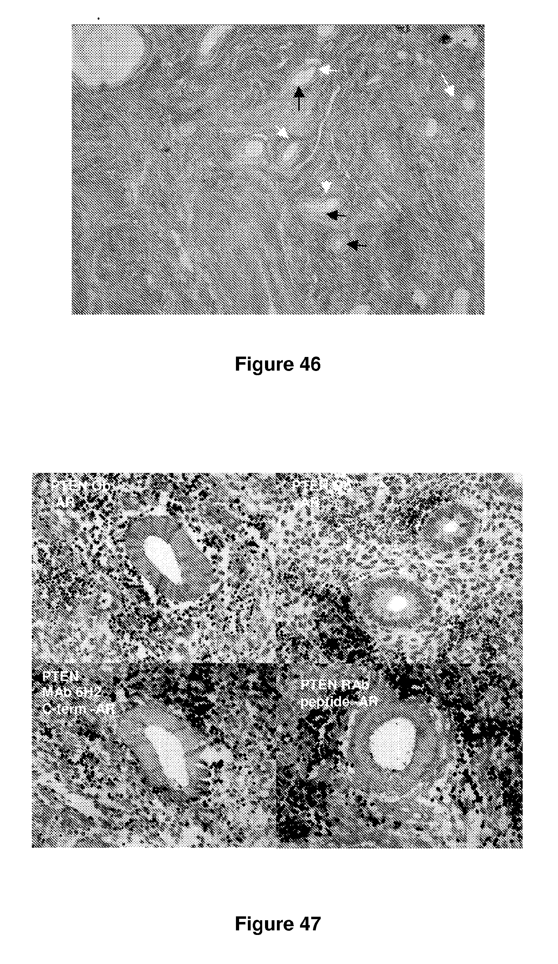

The left panel in figure 46 shows the oligo-antibody staining in glands showing (white arrows) or lacking marks (black arrows). Some of the glands show partial staining losses, in accordance with the aforesaid description of antibodies. It has been magnified 10 times.

### RESULTS OBTAINED WITH PTEN CLONES: 7 -14

At a higher magnification, the comparison of a rabbit's antibody against the same peptide used to select the oligo-antibody, can be observed. Besides, the monoclonal antibody marks the same gland in a serialized section, although with less intensity. Besides, an effect can be observed due to the antigen retrieval in the probe detection. The exposure of tissue to high temperature makes the non-protected DNA to bind to the probe in a non-specific manner. This is evidenced in the tissue stroma marking (tissue appearing between glands), where the staining of the cell nuclei, heterogeneous in the past, is homogenized and the glands nuclei evidence an intense color. The reasons why the marks appear can be due to several causes, either the nuclear proteins bound to DNA or DNA -DNA interactions. For these types of probes, the recovery of the antigen without subsequently eliminating or blocking the non specific binding, is not recommended.

Figure 47 compares staining in typical hyperplasia, in consecutive sections in the left column. The black dots appear due to cell infiltration. The marking of endometrium cells can be seen in cell nuclei as brown spots between glands. Color inside the glands is homogeneous light brown with the oligo-antibodies, the antibodies without treatment for antigen retrieval (-AR). AR: Antigen retrieval; -AR: without treatment for antigen retrieval; PTEN Ob: synthetic PTEN clones numbers 7 and 14; MAb 6H2: Rat Monoclonal Antibody 6H2 (Cascade); PTEN RAb peptide: Antiserum developed for rabbit against the PTEN peptide intertwined with bovine albumin.

### PTENZ SPECIFICITY IN THE MIXTURE OF CLONES 7 AND 14 IN THE ENDOMETRIUM GENETIC MODEL.

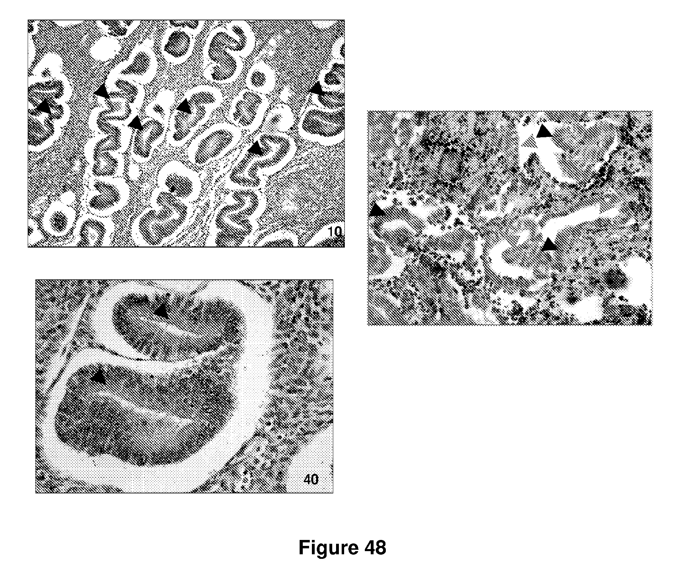

PTEN detection in the endometrium is described at large. The same staining process performed with diaminobenzidine polymers using oligo-antibodies, is also stained with hematoxilin nuclear coloring (dark grey color) and is shown with less than (10x) and more than (40x) magnifications (figure 48).

Oligo-antibodies 7 and 14 are used for endometrium PTEN analysis of control patients. Oligo-antibodies possess biotin in their extreme 5' terminal and are detected with streptavidin-peroxidase and 3-3, di-amino-bencidin tetra hydrochloride (brown) and Hematoxilin is used as contrast staining (blue). Histologies are made in accordance with descriptions and PTEN presence is analyzed in control endometrium (left side at 10 X and 40 X), as well as in endometrium with anomalous growth (right side). Black arrows show PTEN presence in the gland, and the blue ones indicate the areas in the gland that have lost their mark.

Using the genetic model of hyperplasia with anomalous growth it is shown that oligo-antibodies stain areas of hyper atrophied glands, in accordance with reports for PTEN antibodies.

### EXAMPLE 3.12

### HISTOLOGIES USING OLIGO-ANTIBODIES AGAINST CPD1, PEPTIDE A.

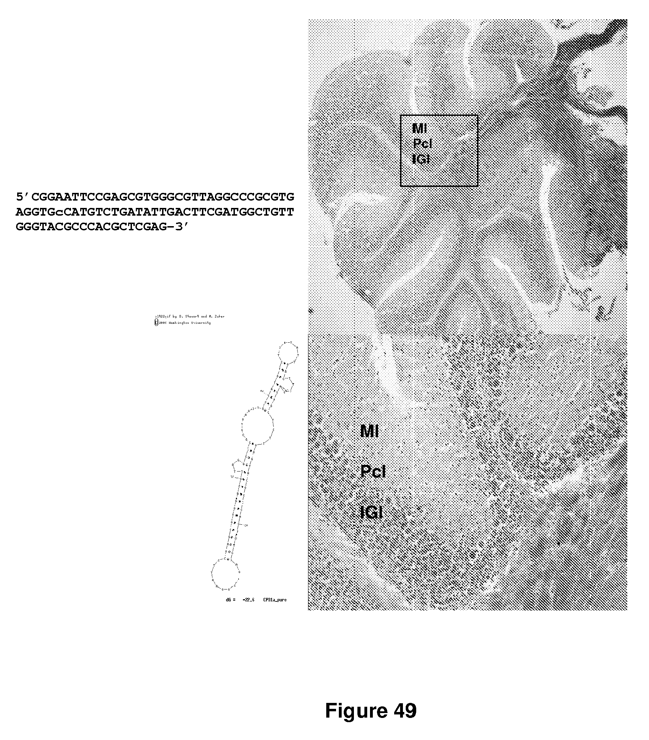

Results obtained with oligo-antibody mixture and the synthetic chain repeat the results. Figure 49 shows Anp32e/Cpd1 detection with the clone at two different magnifications with (sic) the sequence and potential structure.

Figure 49 shows the property of the SIMPLEX protocol products for the recognition of targets, whose properties are kept in the synthetic chain. The image appearing in the first panel shows a murine cerebellum stained for Anp32e/Cpd1 magnified four times. The magnified area is shown in the box and the molecular, Purkinje and internal layers are pointed out in both micrographies. Layers are indicated as ML for the molecular layer, PcL for the Purkinje cell layer and IGL for the internal grain layer. The staining is consistent with the one already described with antibodies. To the left of the panel the oligo-antibody sequence used is shown and the aforementioned structure obtained from the computer programs (http://www.idtdna.com/scitools/Applications/mfold//).

### EXAMPLE 3.13

### CONFOCAL MICROSCOPY OF THE CEREBELLAR DISTRIBUTION OF ANP32E AND OTHER MEMBERS.

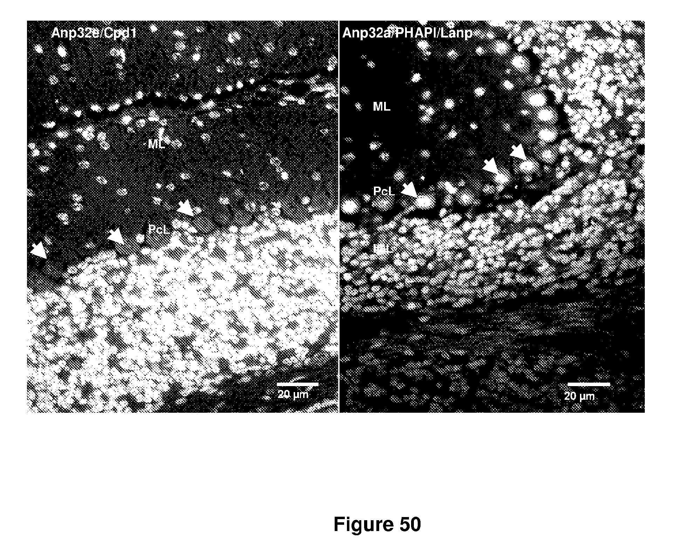

There is an extremely conservative area in the Anp32 protein family. Differences between Anp32e/Cpd1 and other members such as Anp32a/Lanp consist of a single aminoacid changed throughout 23 amino acids. SIMPLEX products are capable of differentiating this change when synthetic peptides of 14 amino acids containing such difference are used. Every oligo-antibody is marked with primers containing fluorescent Cy3. Every marked clone is incubated in histological cerebellum sections and the staining difference can be observed in figure 50.

The fluorescence of the oligo-antibody is detected with a confocal laser microscope. The figure shows the two microscopic observations made in murine cerebellum postnatal 17+days, magnified 200 times. The different layers that make up the histology of the cerebellum of a mouse are indicated as ML for the molecular layer, PcL for the Purkinje cell layer and IGL for the internal grain layer. The most remarkable difference is the nuclear staining that can be observed in the PcL, where the Anp32e detected with the oligo-antibody is very reduced (arrows in the left panel) compared with the remaining Anp32 (arrows in the right panel). Scale bars correspond to 20 microns.

### EXAMPLE 3.14

### RECOGNITION OF DIFFERENT CONSTITUTIONS OF A SAME TARGET PEPTIDE. ANP32E: 66-KDA ISOFORM.

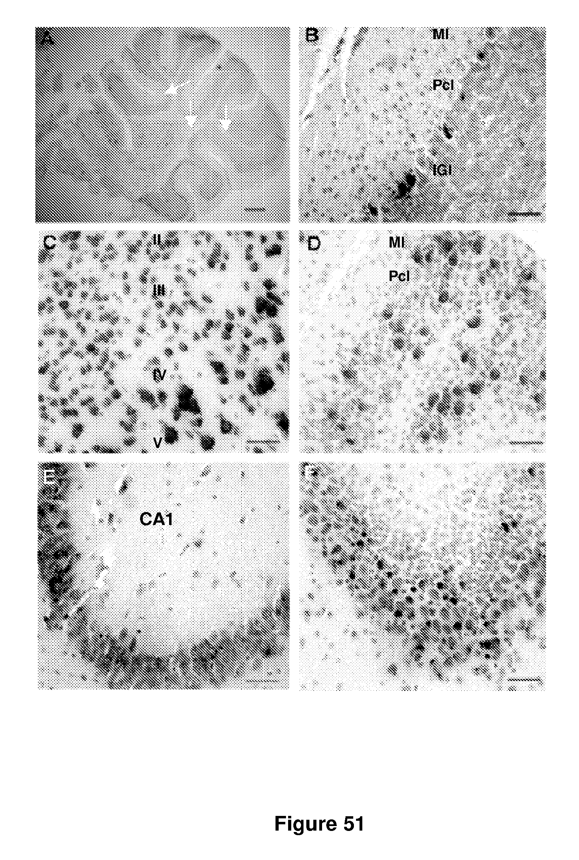

Anp32e has been previously described as 32/34-kDa proteins with nuclear location and as 66-kDa cytoplasmatic proteins and one synapsis isoform with 74-kDa mobility. One of the clones chosen against the peptide a of Cpd1 a is capable of recognizing 66 kDa protein in solution and of purifying it. The same oligo-antibody is used in brain histologies; such microscopic observations appear in figure 51.

Figure 51 shows the distribution of Anp32e protein of 66-kDa on a normal cerebellum (A and B) or the weaver mutant (D), brain cortex (C), hippocampus (E) and the olfactory bulb (F). The scale bar corresponds to 100 µm in A) and 50 µm in the remaining microscopes. The first panel shows the lack of axone and dendrite staining (white arrows). The staining is limited to the cytoplasm of neurons from the internal grain layer and the Purkinje cell layer (B). In the murine cerebellum affected by staggerer mutation (D), the expression of 66-kDa protein is not affected. In panel C, visible brain cortex layers are indicated from II to V; the mark is also observed in cytoplasm of neurons as well as in hippocampus CA1 area (E) or the olfactory bulb.

These results are consistent with the expectations for this protein isoform due to precipitations with the cloned oligo-antibody, reinforcing the capacity of recognizing space target constitution.

### EXAMPLE 3.15

### LIGANDS AGAINST AMINO-TERMINAL PEPTIDES: HUNTINGTIN PROTEIN.

When proteins located in the cell nucleus are studied, it is important to analyze the potential genomic DNA effect as a source of non-specific binding. Having set this goal, the SIMPLEX protocol is applied using a protein known as Huntingtin, which is known to have nuclear and cytoplasmatic location. It is performed with an amplified library and a selected target chosen from the first 12 huntingtin amino acids (MATLEKLMKAFE) bound by the carboxyl end to the albumin and to nitrocellulose.

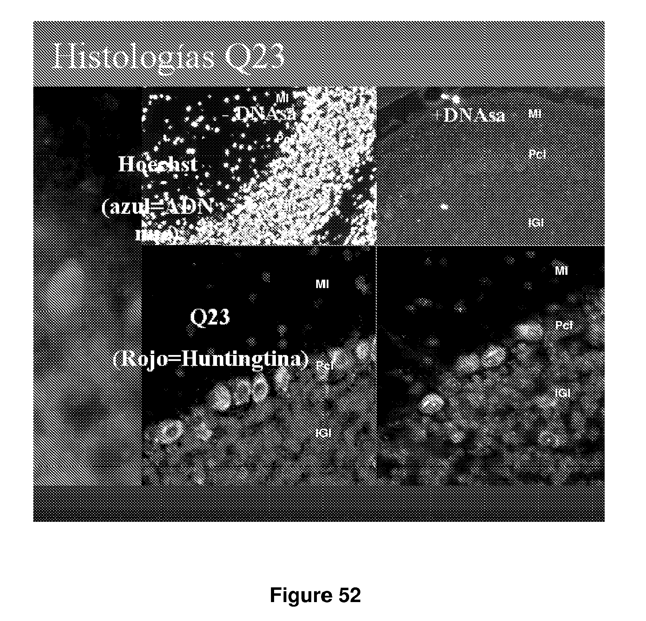

Figure 52 shows the DNA cellular staining with or without DNAse digestion. DNAse treatment makes Hoechst 33258 staining (1:10000, 15 min) disappear, evidencing the absence of DNA (magnified 100 times). At a higher magnification, huntingtin distribution can be observed in murine cerebellum. Layers are indicated as ML for the molecular layer, PcL for the Purkinje cell layer and IGL for the internal grain layer. The oligo-antibody containing Cy3 did not show changes on the topology, and the mark detected in the neuron's nuclei remained red when magnified 200 times (Q23).

### DIFFERENT OLIGO-ANTIBODIES RECOGNIZE THE NUCLEAR HUNTINGTIN PROTEIN DIFFERENTIALLY AND NOT THE CYTOPLASMATIC, IN HISTOLOGICAL SECTIONS.

The protocol is repeated, but the library for the protocols indicated in the Sequence chart as Htt5 is used. Every oligo-antibody is marked with different primers coupled with Cy3 (white) for Q23 and with biotin developed with streptavidin Cy2 (colorless). In this manner, every oligo-antibody is made against the same target, but contrary to expectations, every ligand recognizes a sub-cellular location with differential affinity.

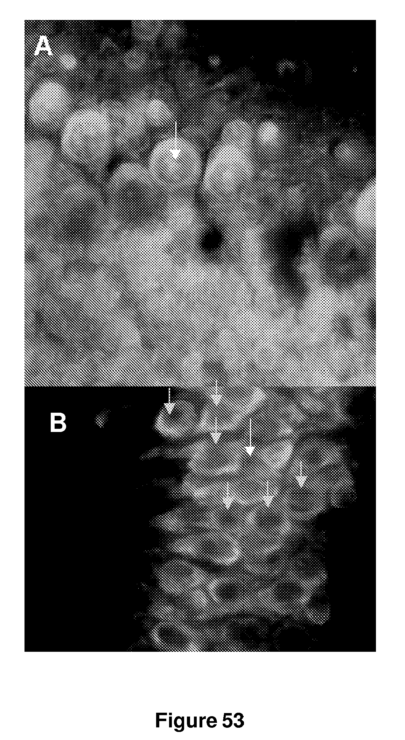

In this assay (figure 53) two oligo-antibodies obtained in separate assays compete with combinatorial libraries having a different structure. It is expected that the mark from both oligo-antibodies overlaps 100%, generating a yellow image. Panel "A" shows a Q23 staining in the Purkinje cell nuclei (green arrow). Staining competition with the oligo-antibody Q23 (Cy3, white) is observed with colored Htt5 detected (Cy2), not shown. As regards target occupation-huntingtin- by the oligo-antibodies Q23 the white mark appears on the cytoplasm and nucleus of Purkinje cell, observed in the cerebellum at P17. This is the case in the neurons' cytoplasm where the whole mark remains. The arrow points out the nuclear location of Huntingtin detected with oligo-antibody Htt5. Such ligand shows a preference for the nuclear protein, competing and preventing the Q23 ligand binding. The white arrow in B shows the loss of Huntingtin detection in neurons' nuclei when incubated in presence of Htt5. The areas appearing in A and B correspond to the cerebellum and hippocampus, respectively, magnified 630 times.

### EXAMPLE 3.16

### SIMULTANEOUS PROTEIN DETECTION USING THREE DIFFERENT OLIGO-ANTIBODIES IN A HISTOLOGICAL SECTION.

Simultaneous detection of multiple targets is possible with oligo-antibodies if they are marked with different fluorochromes. The assay was designed with three proteins, the catalytic subunit of 2 A phosphatase protein (PP2A), Anp32e/Cpd1 (Cpd1) and Huntingtin (Htt), which distribution varies during postnatal development.

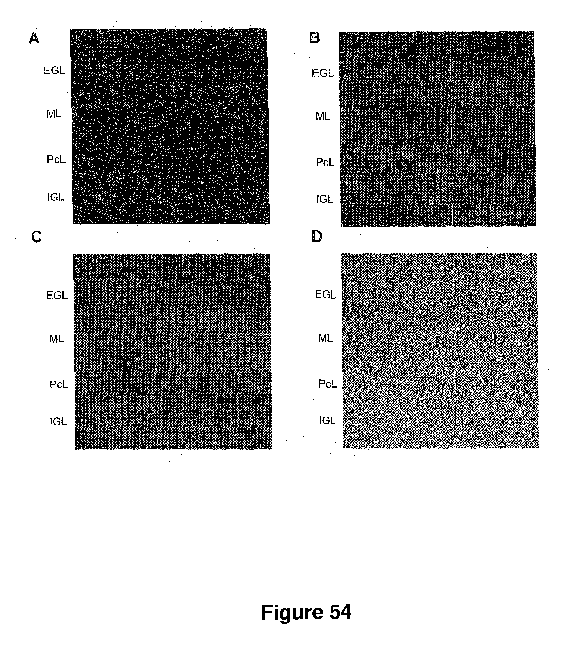

Each oligo-antibody was enlarged independently, using several primers with different fluorescents (figure 54). The oligo-antibody against Cpd1 a with the peptide a was marked with primer nucleotides containing Cy5 (A) Cy3 for primers from the amino end of Huntingtin (B) and Cy2 for primers of PP2A catalytic subunit (C). Oligo-antibodies were separated into a single chain and incubated for three hours. The non-binding oligo-antibody is rinsed and preparations are analyzed with a confocal laser microscope. Each image corresponds to the three-detection position. The direct transmission result is shown in grey (D). Layers making up the cerebellosum cortex are referred to as previously explained, i.e. EGL for the external grain layer, ML for the molecular layer, PcL for the Purkinje cell layer and IGL for the internal grain layer. A comparison is made to show how each oligo-antibody detects its target without interfering with others.

### OLIGO-ANTIBODY AGAINST PP2A IN THE EMBRYO MODEL" D. MELANOGASTER "

This assay reinforces the above example by showing the location of PP2A during the embryo development of Dm and the imaginal disc, showing the location of the eye imaginal disc. The purpose here is to show that the preserved epitope may be detected in this model, reasserting the use of such products in order to analyze protein expression.

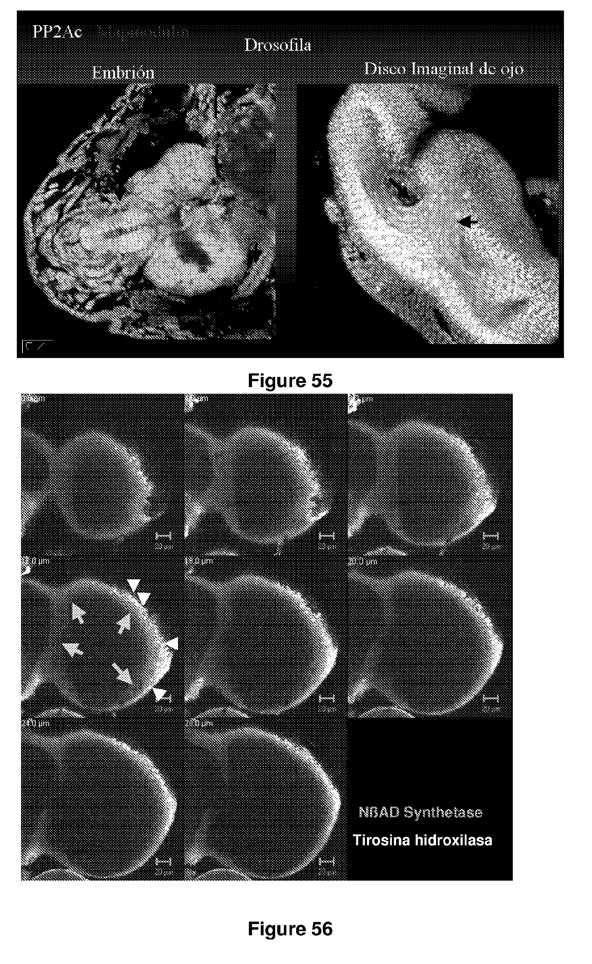

Oligo-antibodies show staining differences (figure 55), thus evidencing the specificity in the recognition of the catalytic subunit of 2A phosphatase protein (PP2A), (PP2Ac, indicated with black arrows). Nuclear phosphatase expression is intense throughout these development periods. The chart on the left shows the staining of an embryo where the PP2Ac is located in the pharynx muscle (thick white arrow), while the chart of the imaginal disc on the right also shows the nuclear staining over the same development period during the metamorphosis. Both images are magnified 630 times in a 1 µm thick focal plane.

### EXAMPLE 3.17

### N-β-ACETYL DOPAMINE TRANSFERASE OR NDAB SYNTHASE IN DROSOPHILA MELANOGASTER BRAINS.

Ebony: This is the name of the gene responsible for the coding of N-β-Acetyl dopamine transferase or NBAD synthase enzyme. Clones of the SIMPLEX products against this enzyme were used and assayed in dissected fly brains. The staining is done with the oligo-antibody (Cy3, gray staining) and the antibodies against tyrosine hydroxylase (TH) detected with antibodies (Alexa-488, white staining). The distribution of the NBADs enzyme can be seen in several sections of confocal planes along an adult fly brain hemisphere. The resulting staining coincides with the location of glial cells (gray) although it can also be seen in neuronal type cells (Figure 56).

The brains were dissected, paraformaldehyde-fixed, detergent-permeabilized and stained using the NBADs oligo-antibody (clone 3) amplified with primers containing Cy3. The TH is detected using conventional antibodies and is located in dopaminergic neurons. The NBADs staining is observed in glial cells (gray) and only in some dopaminergic neurons (white arrows). The heights of the confocal planes are shown on top of each panel and the bar represents 20 micrometers.

### EXAMPLE 3.18

### SPARC IN THE EPIDERMIS AND IN THE DROSOPHILA MELANOGASTER FLY

The SPARC protein expression can be found in the epidermis and in the hair follicles. This particular location of SPARC is used to corroborate the identity of the target recognized by the oligo-antibody. Hence, epidermis sections of "Ratus norvergicus" were taken in order to stain them with the oligo-antibody and to develop them by the peroxidase activity according to the descriptions above.

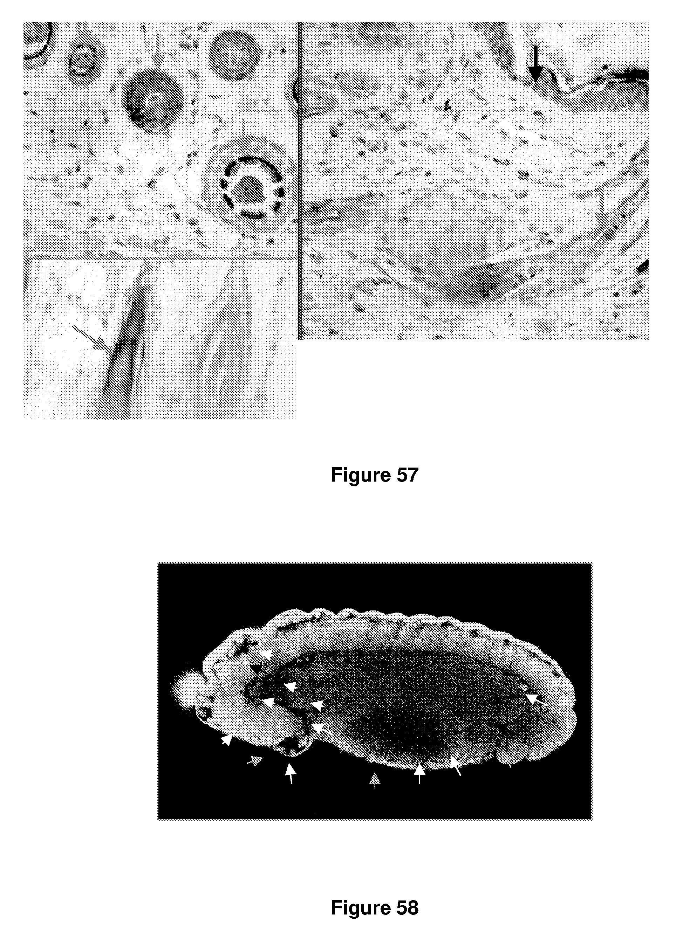

The microscopy shows the distribution of SPARC expression in rat epidermis. Greater concentrations can be observed in the hair follicles (gray arrow) and in the epidermis (black arrow, figure 57). The magnification is 200 times.

The oligo-antibody was assayed in fly embryos, where SPARC protein expression is preserved. The retardation gels had shown that the oligo-antibody produces a remarkable retard-effect in embryos.

In figure 58, embryos in the stage 12 are shown in a confocal plane. The fluorescence is shown in white and the gray arrows show the SPARC expression in isolated cells, possibly hemocytes. The black arrows show the presence of SPARC in the CNS and the red ones show its expression in the epidermis.

### EXAMPLE 3.19

### OLIGO-ANTIBODIES FOR PROTEIN LOCATION THROUGH TRANSMISSION ELECTRON MICROSCOPY (TEM)

The purpose of this example is to illustrate the detection capacity of the products obtained by the SIMPLEX method. This is performed by the determination of the subcellular location of the selected proteins using the electronic microscope. Male adult mice are subjected to intra-cardiac perfusion with saline solution followed by 4% paraformaldehyde in 0.1 M of cacodylate. The brains extracted are sectioned with a vibratome in 40 µm thick sections and used for the detection of the Anp32e/Cpd1 or huntingtin protein. Using the property of electro-density of diaminobenzidine polymers, the oligo-antibodies were developed with peroxidase. The sections were extensively rinsed and Osmium contrasted for one hour (1 % OsO4 in phosphate buffer), dehydrated in gradual series of ethanol and propylene oxide, soaked in Spurr resin, and polymerized for 48 hours at 60ºC. Ultra thin sections were obtained with a Sorvall Porter-Blum MT2-B ultra-microtome, and contrasted with uranyl acetate and Reynolds' lead citrate to be examined by a JEÖL 1200 EX2 transmission microscope at 85 kV.

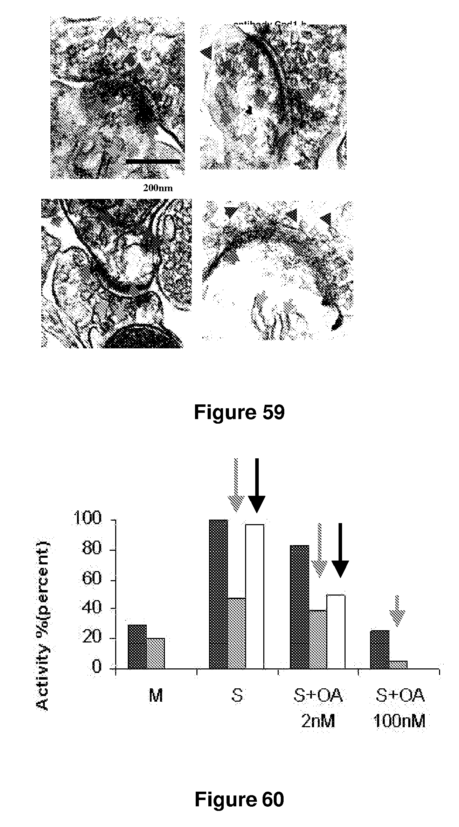

Figure 59 shows the images of the electronic density of the cerebellum sections obtained by TEM. The stainings were done using conventional antibodies (against the b-peptide or Cpd1-b) or oligo-antibodies against the a-peptide (Cpd1-a), both antibodies recognizing Anp32e/Cpd1 protein and the amino-terminal fragments of huntingtin (Htt5). The peroxidase staining shows that the location of Cpd1 is the same using the antibody or the oligo-antibody. Both present presynaptic vesicles labels (black arrows), postsynaptic densities (dark gray arrows) and endosome membranes (gray arrows). This labeling could also be observed with the oligo-antibody Htt5 for huntingtin.

### EXAMPLE 3.20

### THE PURIFIED ANP32E/CPD1 PROTEIN INHIBITS PP2A AND THIS ACTIVITY IS BLOCKED BY THE CPD1-A OLIGO-ANTIBODY.

Figure 60 describes the Anp32e/Cpd1 inhibitory property relative to the phosphatase activity of PP2A. The products of protein purification with SELEX products are tested for the Cpd1-a case, analizyng its inhibitor activity. Moreover, the product of SIMPLEX procedure can block the activity of Anp32e/Cpd1 protein.

Another way of characterizing the purified protein with the Cpd1-a oligo-antibody is analyzing the Anp32e/Cpd1 property as inhibitor of the phosphatase activity of PP2A. In order to do so, subcellular fractions of murine cerebellum, where PP2A activity can be found, were analyzed. The assay consists in adding the Anp32e/Cpd1 protein to a concentration of 10 nM (Cpd1 in gray) and measuring the phosphatase activity in the microsomal (M) and synaptic (S) subcellular fraction. The activity is measured using the phosphorylase protein marked with ³²PO₄ through the casein kinase II (CKII). This phosphoprotein is a substrate of protein phosphatases PP2A and PP1 and what is measured is the amount of phosphate released by them. The activities are differentiated by the okadaic acid (OA), a phosphatase inhibitor, used at a concentration of 2 nM for the PP2A and of 100 nM for both. The table shows the results expressed as a percentage of the maximum phosphatase activity, achieved in this case. by the synaptic fraction. When the protein Anp32e/Cpd1 is preincubated with the oligo-antibody Cpd1-a (1µM), the loss of the inhibitor activity can be observed (Cpd1+Ob), recovering the maximum of its activity. On the other hand, this is the same percentage of activity that is lost with the okadaic inhibitor at 2nM, i. e., the activity of the PP2A.

This result emphasizes the use of these reagents as enzymatic activity inhibitors, opening up the possibility for a new application in pharmacogenetics.

### EXAMPLE 3.21

### THE SIMPLEX LIBRARIES ARE APPLICABLE TO PEPTIDE AND PROTEIN ARRANGEMENTS.

The diagram represents the nitrocellulose with the peptides (10 nanomoles) or proteins arranged in arrays. The spaces with free binding sites of the membrane are covered with albumin and the whole membrane is presented to the amplified combinatorial library (300 µl of PCR -25 cycles- in 600 µl of PBS-BSA 5%). The bound ligands are eluted (OHNa 0,15 N 30 seg., neutralizes 0,15N CIH) and used to amplify by PCR with marked primers (20 cycles). The specificity of the obtained reagents is shown in complex protein specimens as cerebellar homogenates. The mixture of oligo-antibodies is presented to homogenates separated according to molecular size in Western blot assays. As control, the target is analyzed using the "dot blot" technique. The right panel shows that each mixture is consistent with the description given for its molecular weight, and it is also capable of recognizing the original receptor target.

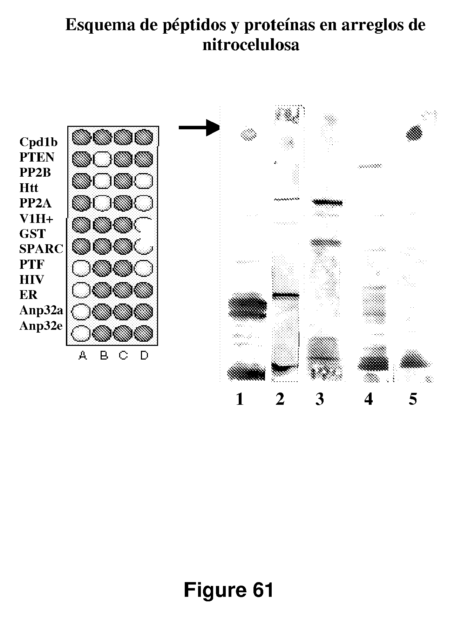

Each membrane is blocked with PBS-BSA 5% and incubated overnight with the PCR products heated at 95ºC for 5 minutes and cooled during the dilution of these ligands (200 nM final) 1: 5 with PBS buffer-5% BSA at 4ºC. The bound ligands are detected and developed with the streptavidin-alkaline phosphatase system. Figure 61 shows the lanes that correspond to the detection of the oligo-antibody with peptide corresponding to Anp32e/Cpd1 (1), PTEN (2), PP2B (3), Huntingtin (4) y PP2A (5). The control peptide were applied in the form of drops or "dot" in the upper part of the lane, except for the huntingtin, which can be observed at the bottom of the run. Each oligo-antibody mixture is capable of recognizing its target contained in the protein, showing the detection of differences in molecular size in the band detected by the Western blot.

### EXAMPLE 4.1

### RECOGNITION OF THE LIBRARIES WITH COMPLEX MIXTURES OF TARGETS: OPTIMIZATION OF THE LIBRARY

The assay is done using a cerebellar protein homogenate placed in the form of drops over a defined dimension of nitrocellulose (0.12 mm²). The membrane is presented to a library of 1.2 ml (400 µl of PCR in 800 µl of PBS-5% BSA). This amount of proteins allows us to isolate in a single selection enough quantity of ligands to examine a wide range of cerebellar proteins in Western blot assays. The specificity against the mixture is proven by the competence with the double amount of proteins absorbed in the membrane; only an intense band and a weak band of greater molecular weight are detected. Amplifications after the selection should be avoided, because these affinities imply a loss in variability. For example, an amplification of 1:100 used in an amplification of PCR to be incubated with the same cerebellar protein mixture only recognizes an intense band with a molecular weight of 30 kDa.

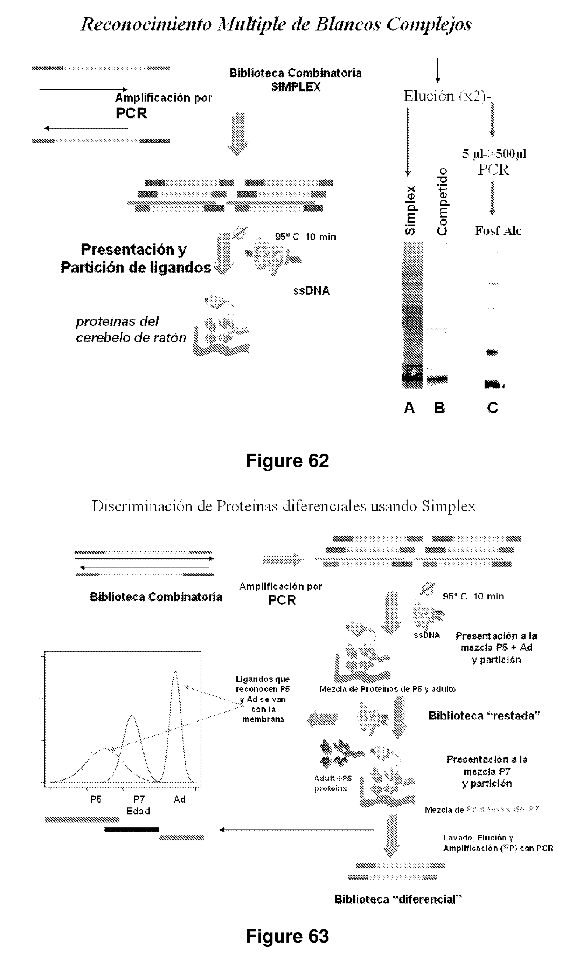

Figure 62 shows ligands obtained according to the diagram illustrated on the left, using murine cerebellar proteins on postnatal day 7. The ligands obtained undergo 6 PCR cycles in presence of biotin primers and are assayed in Western blot assays. The PCR mixture (SIMPLEX) can detect a broad range of proteins that can be found in the cerebellum on postnatal day 7 (A). The adjoining lane shows the other half of the preincubated mixture exposed to the protein mixture overnight (competed) and then assayed by the Western blot (B). The amplification with 25 PCR cycles of 1% of the obtained ligands, when the Western blot is tested (alkaline phosphatase) produces the detection of a single band, showing a huge loss in the variability of the targets (C).

### EXAMPLE 4.2

### RECOGNITION OF THE LIBRARIES WITH COMPLEX MIXTURES OF TARGETS: SUBSTRACTION OF LIGANDS.

The purpose of the assay is to obtain ligands against proteins, the expression of which is exclusive of the state of a tissue. As a model, we use the cerebellum where only the 3% of all the expressed genes have a transitory expression during its development. Taking an age as target (P7), the chosen proteins are reduced by subtracting the early ages and the adult age where other cellular processes take place.

The diagram in figure 63 shows the steps of the procedure that was carried out to recognize differential targets among thousands of possible targets. The amplified library is presented during the whole night to the protein mixtures from adult and postnatal day 5 cerebellums. These are supported in membranes with a surface that is ten times the surface used with the target proteins of age P7. The library that did not recognize any target in the membranes of the subtraction, is presented during three hours to a membrane of 0.12 mm² containing proteins P7. After washing the membrane ligands, these are eluted and marked with ³²P through PCR.

### OBTAINING DIFFERENTIAL LIGANDS DURING CEREBELLUM DEVELOPMENT

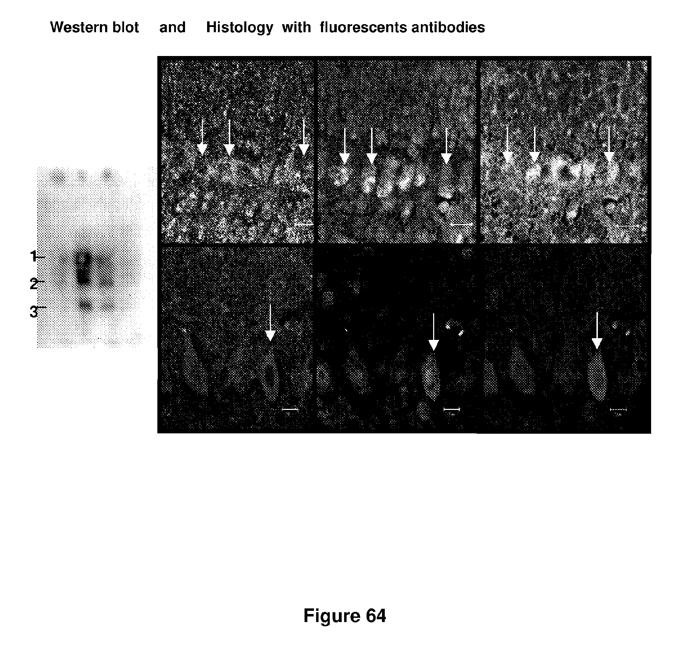

The ligands obtained according to the previous differential process are assayed using Western blot assays. Three of the differential bands obtained in the Western blot are eluted individually and amplified using primers with different fluorescent agents. Each oligo-antibody mixture is presented in histological sections of postnatal day 7 and adult cerebellums, showing a differential staining for each oligo-antibody similar to the detection produced in the membrane with Western blot. The topological staining differential of each target, plus the difference in molecular size, allows us to conclude that each band corresponds to different proteins with differential expression at P7 and not only to a product of the degradation of a single protein with differential expression.
The Western blot assay in figure 64 was done with the same proteins as the ones used to present the target. 50 µg of proteins corresponding to each postnatal age were separated in a SDS-PAGE of 10%, the proteins transferred to nitrocellulose and incubated overnight with the PCR selection amplified 10 cycles in presence of 32P. The autoradiography shows the distribution of the enriched mark in the lane that contains the proteins from the murine cerebellums on postnatal day 7 (P7). The control of protein load was done with the marketed antibody against the phopsphatase PP1 (control). The bands were eluted and amplified with a fluorescent primer as Cy2 in band 1, Cy3 in band 2 and Cy5 in band 3. The mixture of the three bands is incubated in the same histological section and analyzed during the development comparing P7 and Adult (left bottom panel). Both images are the overlapping of the three oligo-antibodies obtained with a confocal laser microscope, which are shown individually at P7 (right panel). A co-location of the three marks does not exist, having the nuclear location for band 3; the col-location of band 1, and 2 is cytoplasmatic in the Purkinje cell layer (PCL), and individual in the molecular layer (ML).

### OBTAINING DIFFERENTIAL LIGANDS IN A BREAST TUMOR CELL LINE.

Another model used includes a breast tumor line (C7-2-HT), which injected subcutaneously in solid form progresses to a primary tumor or lung metastases. The strategy is to find differential proteins expressed in the primary tumor and the selection is made by subsequently subtracting the proteins from the lung metastasis. The obtained ligands are amplified in the presence of ³²P-dCTP and with fluorescent primers (Cy3). The assay with primary tumor proteins and radioactive ligands detect a single protein of 40 kDa which is clearly expressed in the glands formed by the cells in the primary tumor (Left panel, figure 62).

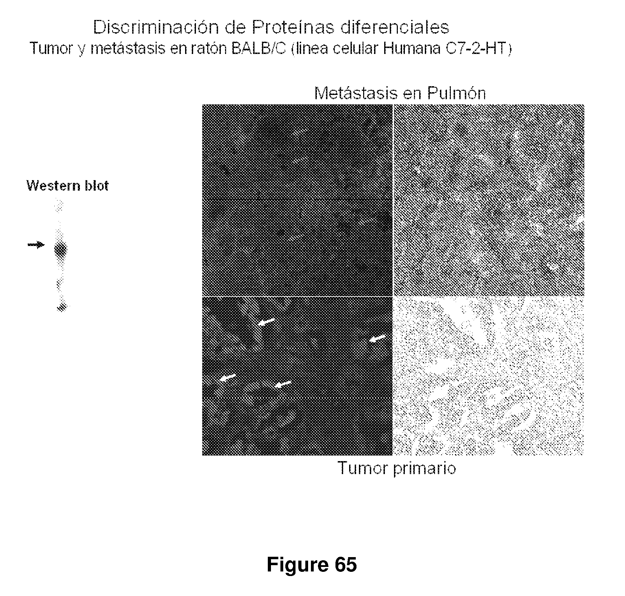

The amplified ligands are presented to a 9 cm² nitrocellulose membrane containing proteins obtained from lung metastasis. Two milliliters of PBS-BSA 5% plus 1 ml of preincubaed PCR (³²P-dCTP) are presented. The proteins from the primary tumor are presented to the SIMPLEX library in a 0.2 cm² membrane. The ligands obtained from the selection are amplified 22 PCR cycles in the presence of ³²P-dCTP with biotine primers (1ml of PCR) and presented to a lane of proteins separated by molecular size on SDS-PAGE. Figure 65 shows in the X-ray a 45 kDa protein after one night of incubation at 4ºC (arrow on left panel). The PCR products are separated in single strain and used in paraffin sections under exactly the same conditions for primary tumor and metastasis (right panel). After developing the presence with Cy3-bound streptoavidine, the sections are analyzed in a confocal laser scanning microscope (right panel). The presence is evident in the primary tumor (white arrows) and is not present in the metastasis (grey arrows).

SEQUENCE LISTING

<110>  Radrizzani, Martin
       Halitus Instituto Medico S.A.

<120>  SELECTION PROCEDURE IMPLEMENTED BY EXCESS LIGANDS

<130>  EP-582

<150>  P20060104421
<151>  2006-10-06

<160>  62

<170>  PatentIn version 3.3

<210>  1
<211>  21
<212>  DNA
<213>  artificial sequence

<220>
<223>  Primer

<400>  1
cggaattccg agcgtgggcg t                                                21

<210>  2
<211>  16
<212>  DNA
<213>  artificial sequence

<220>
<223>  Primer

<400>  2
ctcgagcgtg ggcgta                                                      16

<210>  3
<211>  87
<212>  DNA
<213>  artificial sequence

<220>
<223>  Template

<220>
<221>  misc_feature
<222>  (22)..(71)
<223>  n is a, c, g, or t

<400>  3
cggaattccg agcgtgggcg tnnnnnnnnn nnnnnnnnnn nnnnnnnnnn nnnnnnnnnn      60

nnnnnnnnnn ntacgcccac gctcgag                                          87

<210>  4
<211>  19
<212>  DNA
<213>  artificial sequence

<220>
<223>  Primer

<400>  4
tgcaggccgc ggggaagga                                                    19


<210>  5
<211>  91
<212>  DNA
<213>  artificial sequence

<220>
<223>  Template


<220>
<221>  misc_feature
<222>  (20)..(72)
<223>  n is a, c, g, or t

<400>  5
tgcaggccgc ggggaaggan nnnnnnnnnn nnnnnnnnnn nnnnnnnnnn nnnnnnnnnn      60

nnnnnnnnnn nntccttccc cgcggcctgc a                                      91


<210>  6
<211>  98
<212>  DNA
<213>  artificial sequence

<220>
<223>  Template


<220>
<221>  misc_feature
<222>  (20)..(79)
<223>  n is a, c, g, or t

<400>  6
tgcaggccgc ggggaaggan nnnnnnnnnn nnnnnnnnnn nnnnnnnnnn nnnnnnnnnn      60

nnnnnnnnnn nnnnnnnnnt ccttccccgc ggcctgca                               98


<210>  7
<211>  19
<212>  DNA
<213>  artificial sequence

<220>
<223>  Primer

<400>  7

gacacacgcc tctatcata                                                    19


<210>  8
<211>  20
<212>  DNA
<213>  artificial sequence

<220>
<223>  Primer

<400>  8
tccattcaca gaggttttga                                                   20


<210>  9
<211>  119
<212>  DNA
<213>  artificial sequence

<220>
<223>  Template


<220>
<221>  misc_feature
<222>  (21)..(100)
<223>  n is a, c, g, or t

<400>  9
tccattcaca gaggttttga nnnnnnnnnn nnnnnnnnnn nnnnnnnnnn nnnnnnnnnn      60

nnnnnnnnnn nnnnnnnnnn nnnnnnnnnn nnnnnnnnnn tatgatagag gcgtgtgtc       119


<210>  10
<211>  87
<212>  DNA
<213>  artificial sequence

<220>
<223>  Specific sequence of the oligonucleotide that recognizes the PTEN
       protein

<400>  10
cggaattccg agcgtgggcg tccccacgca agatttgtag gtcagtatat gtcgggcttg      60

tttatgtgcc gtacgcccac gctcgag                                          87


<210>  11
<211>  87
<212>  DNA
<213>  artificial sequence

<220>
<223>  Specific sequence of the oligonucleotide that recognizes the PTEN
       protein

<400>  11
cggaattccg agcgtgggcg tctagcccac ccataaatct cccaatcggg gtgcagttag      60

aagttgacgt ctacgcccac gctcgag                                          87

<210>  12
<211>  87
<212>  DNA
<213>  artificial sequence

<220>
<223>  Specific sequence of the oligonucleotide that recognizes the PTEN
       protein

<400>  12
cggaattccg agcgtgggcg ttagacacgc gacgggctca ttgctttatt gtgataatct     60

atcttcgttg gtacgcccac gctcgag                                          87

<210>  13
<211>  87
<212>  DNA
<213>  artificial sequence

<220>
<223>  Specific sequence of the oligonucleotide that recognizes the PTEN
       protein

<400>  13
cggaattccg agcgtgggcg tctgtggacg ccttaccttt tgcgatccag cttgttccgg     60

tgttgtcgtg gtacgcccac gctcgag                                          87

<210>  14
<211>  87
<212>  DNA
<213>  artificial sequence

<220>
<223>  Specific sequence of the oligonucleotide that recognizes the PTEN
       protein

<400>  14
cggaattccg agcgtgggcg tgtttgggag taaacacgac acagcgcggc aggtcttaca     60

cggctcttct ctacgcccac gctcgag                                          87

<210>  15
<211>  87
<212>  DNA
<213>  artificial sequence

<220>
<223>  Specific sequence of the oligonucleotide that recognizes the PTEN
       protein

<400>  15
cggaattccg agcgtgggcg tggtcatacc gcgcctatcg aactcgccac tcgcgtgcag     60

ctctgtgtag gtacgcccac gctcgag                                          87

<210>  16
<211>  87
<212>  DNA
<213>  artificial sequence

<220>
<223>  Specific sequence of the oligonucleotide that recognizes the PTEN
       protein

<400>  16
cggaattccg agcgtgggcg tcagcccatt tcttacgaac gtcccatgtg catctccgag     60

acattaccgg gtacgcccac gctcgag                                          87

<210>  17
<211>  87
<212>  DNA
<213>  artificial sequence

<220>
<223>  Specific sequence of the oligonucleotide that recognizes the PTEN
       protein

<400>  17
cggaattccg agcgtgggcg ttaggcccgc gtgaggtgac atgtctgata ttgacttcga     60

tggctgttgg gtacgcccac gctcgag                                          87

<210>  18
<211>  87
<212>  DNA
<213>  artificial sequence

<220>
<223>  Specific sequence of the oligonucleotide that recognizes the PTEN
       protein

<400>  18
cggaattccg agcgtgggcg tgtcggcccc agcgaggtga taagcgaatc cgaatgcagg     60

ttaatgatgt gtacgcccac gctcgag                                          87

<210>  19
<211>  87
<212>  DNA
<213>  artificial sequence

<220>
<223>  Specific sequence of the oligonucleotide that recognizes the PTEN
       protein

<400>  19
cggaattccg agcgtgggcg tggggcgccc ataagcgagg ggatgcggag gttctgtaca     60

cgtgtgcttg gtacgcccac gctcgag                                          87

<210>  20
<211>  87
<212>  DNA
<213>  artificial sequence

<220>
<223>  Specific sequence of the oligonucleotide that recognizes the PTEN
       protein

<400>  20
cggaattccg agcgtgggcg tgagccctaa acacaagtcc gcaggggtgt ggtaatattc     60

gcagttgtgt gtacgcccac gctcgag                                         87


<210>  21
<211>  87
<212>  DNA
<213>  artificial sequence

<220>
<223>  Specific sequence of the oligonucleotide that recognizes the PTEN
       protein

<400>  21
cggaattccg agcgtgggcg ttgcccacgt attagtgtta atctttactt ggaaagcggt     60

gcacgtccca gtacgcccac gctcgag                                         87


<210>  22
<211>  87
<212>  DNA
<213>  artificial sequence

<220>
<223>  Specific sequence of the oligonucleotide that recognizes the PTEN
       protein

<400>  22
cggaattccg agcgtgggcg tcgagtgaca gtcccacgca atatccaggg atctaacaac     60

atccgctgct gtacgcccac gctcgag                                         87


<210>  23
<211>  87
<212>  DNA
<213>  artificial sequence

<220>
<223>  Specific sequence of the oligonucleotide that recognizes the PTEN
       protein

<400>  23
cggaattccg agcgtgggcg ttgcccacat tcggttgtcg agtgtattga agtggaacgg     60

tccttacatg gtacgcccac gctcgag                                         87

<210>  24
<211>  87
<212>  DNA
<213>  artificial sequence

<220>
<223>  Specific sequence of the oligonucleotide that recognizes the PTEN
       protein

<400>  24
cggaattccg agcgtgggcg ttgagccccg cgagctactt ggctgtagtc tgctggttca     60

tcacgcgtcg gtacgcccac gctcgag                                         87


<210>  25
<211>  87
<212>  DNA
<213>  artificial sequence

<220>
<223>  Specific sequence of the oligonucleotide that recognizes the PTEN
       protein

<400>  25
cggaattccg agcgtgggcg tgaccacgcg acttggtaat cgactcttat attgacgcag     60

cttagaggtt gtacgcccac gctcgag                                         87


<210>  26
<211>  87
<212>  DNA
<213>  artificial sequence

<220>
<223>  Specific sequence of the oligonucleotide that recognizes the PTEN
       protein

<400>  26
cggaattccg agcgtgggcg taccgaaaca tattcctcaa attgcgtacc atggcggctg     60

tgctgggcgt ctacgcccac gctcgag                                         87


<210>  27
<211>  87
<212>  DNA
<213>  artificial sequence

<220>
<223>  Specific sequence of the oligonucleotide that recognizes the PTEN
       protein

<400>  27
cggaattccg agcgtgggcg tgttggagat tcgacgcagt tatattctca gctaatggtt     60

cagcgcacca gtacgcccac gctcgag                                         87

<210>  28
<211>  87
<212>  DNA
<213>  artificial sequence

<220>
<223>  Specific sequence of the oligonucleotide that recognizes the PTEN
       protein

<400>  28
cggaattccg agcgtgggcg tcgcccactc ccatgggtac tttgtatagt tccctttcat      60

acggttgcct gtacgcccac gctcgag                                          87


<210>  29
<211>  98
<212>  DNA
<213>  artificial sequence

<220>
<223>  Specific sequence of the oligonucleotide that recognizes the PTEN
       protein

<400>  29
tgcaggccgc ggggaaggac aacacaatat catgagcaca tcttccatca ggatcgccgc      60

ggctgctacc tgcttacgct ccttccccgc ggcctgca                              98


<210>  30
<211>  87
<212>  DNA
<213>  artificial sequence

<220>
<223>  Specific sequence of the oligonucleotide that recognizes the GST
       protein

<400>  30
cggaattccg agcgtgggcg ttagcccata atgtggtgca ttattcgcta gatttcgggg      60

atacttttca gtacgcccac gctcgag                                          87


<210>  31
<211>  87
<212>  DNA
<213>  artificial sequence

<220>
<223>  Specific sequence of the oligonucleotide that recognizes the GST
       protein

<400>  31
cggaattccg agcgtgggcg tacccaacag ccatcgaagt caatatcaga catgtcacct      60

cacgcgggcc ttacgcccac gctcgag                                          87


<210>  32

<211>  87
<212>  DNA
<213>  artificial sequence

<220>
<223>  Specific sequence of the oligonucleotide that recognizes the PTEN
       protein

<400>  32
cggaattccg agcgtgggcg taggccctcg cgaggtcagg ttgttaattg gggtatttta      60

cacttacgca gtacgcccac gctcgag                                          87


<210>  33
<211>  87
<212>  DNA
<213>  artificial sequence

<220>
<223>  Specific sequence of the oligonucleotide that recognizes the
       NBADs protein

<400>  33
cggaattccg agcgtgggcg tgagccacgc gagatttgtg gcgcactgca actaaacact      60

caatctgtcc gtacgcccac gctcgag                                          87


<210>  34
<211>  87
<212>  DNA
<213>  artificial sequence

<220>
<223>  Specific sequence of the oligonucleotide that recognizes the
       NBADs protein

<400>  34
cggaattccg agcgtgggcg tccgacgcgt gatgaaccag cagactacag ccaagtagct      60

cgcggggctc atacgcccac gctcgag                                          87


<210>  35
<211>  87
<212>  DNA
<213>  artificial sequence

<220>
<223>  Specific sequence of the oligonucleotide that recognizes the
       SPARC protein

<400>  35
cggaattccg agcgtgggcg tatgcccacg taaggtttta ggtctccagc cgtaattgta      60

tacgaattct gtacgcccac gctcgag                                          87


<210>  36
<211>  87

<212>  DNA
<213>  artificial sequence

<220>
<223>  Specific sequence of the oligonucleotide that recognizes the
       Lysozyme protein

<400>  36
cggaattccg agcgtgggcg tctagcccac aaccatgata cgtaccttta tgttcatctt      60

ccgggcttta ctacgcccac gctcgag                                          87


<210>  37
<211>  87
<212>  DNA
<213>  artificial sequence

<220>
<223>  Specific sequence of the oligonucleotide that recognizes the
       Lysozyme protein

<400>  37
cggaattccg agcgtgggcg tggaacgccc ttacagatgg agtgtaataa tacgtttggt      60

gccgaactag gtacgcccac gctcgag                                          87


<210>  38
<211>  87
<212>  DNA
<213>  artificial sequence

<220>
<223>  Specific sequence of the oligonucleotide that recognizes the
       Lysozyme protein

<400>  38
cggaattccg agcgtgggcg tgcgcacggg ctaatatgtt aaacttttgg ggtatcacct      60

agatactagt atacgcccac gctcgag                                          87


<210>  39
<211>  87
<212>  DNA
<213>  artificial sequence

<220>
<223>  Specific sequence of the oligonucleotide that recognizes the
       Anp32e34-kDa protein

<400>  39
cggaattccg agcgtgggcg ttaggcccgc gtgaggtgac atgtctgata ttgacttcga      60

tggctgttgg gtacgcccac gctcgag                                          87


<210>  40
<211>  87
<212>  DNA

<213>  artificial sequence

<220>
<223>  Specific sequence of the oligonucleotide that recognizes the
       Anp32e protein

<400>  40
cggaattccg agcgtgggcg ttgcgcccac cttagatcaa tgtcagttgc tcatcgtcac      60

cagcttgtca ttacgcccac gctcgag                                          87


<210>  41
<211>  87
<212>  DNA
<213>  artificial sequence

<220>
<223>  Specific sequence of the oligonucleotide that recognizes the
       Anp32e 66-kDa protein

<400>  41
cggaattccg agcgtgggcg tcatgcgtaa agctcaaaac catctgtgga acatttatca      60

gatctaagtc ctacgcccac gctcgag                                          87


<210>  42
<211>  87
<212>  DNA
<213>  artificial sequence

<220>
<223>  Specific sequence of the oligonucleotide that recognizes the
       Anp32a protein

<400>  42
cggaattccg agcgtgggcg ttgtaacacg aacacgggag tttctggatg gtcttatgaa      60

aggtagggcg gtacgcccac gctcgag                                          87


<210>  43
<211>  87
<212>  DNA
<213>  artificial sequence

<220>
<223>  Specific sequence of the oligonucleotide that recognizes the
       Anp32a protein

<400>  43
cggaattccg agcgtgggcg tgagccaacc tcggtattgt cattagcgta gatggtggat      60

tagccgctac atacgcccac gctcgag                                          87


<210>  44
<211>  87
<212>  DNA
<213>  artificial sequence

<220>
<223>  Specific sequence of the oligonucleotide that recognizes the
      Anp32a protein

<400>  44
cggaattccg agcgtgggcg taagcccagc agcgatatga caaatttaac gctctgctga      60

ccagccatgt gtacgcccac gctcgag                                          87


<210>  45
<211>  87
<212>  DNA
<213>  artificial sequence

<220>
<223>  Specific sequence of the oligonucleotide that recognizes the
      Anp32a protein

<400>  45
cggaattccg agcgtgggcg tgtttgacgc ccgtaatgta agagtcatac ttcgctggta      60

aatgctcgtg gtacgcccac gctcgag                                          87


<210>  46
<211>  87
<212>  DNA
<213>  artificial sequence

<220>
<223>  Specific sequence of the oligonucleotide that recognizes the
      Estrogen Receptor protein (ER1)

<400>  46
cggaattccg agcgtgggcg ttgcccacct tcgactttct tatcacggat ccgctttaca      60

tctggccgcg gtacgcccac gctcgag                                          87


<210>  47
<211>  87
<212>  DNA
<213>  artificial sequence

<220>
<223>  Specific sequence of the oligonucleotide that recognizes the
      Estrogen Receptor protein (ER1)

<400>  47
cggaattccg agcgtgggcg tccgttacac acccagctgc tggactacat catttgaaat      60

acatggtttg gtacgcccac gctcgag                                          87


<210>  48
<211>  87
<212>  DNA
<213>  artificial sequence

<220>
<223>  Specific sequence of the oligonucleotide that recognizes the
       Estrogen Receptor protein (ER1)

<400>  48
cggaattccg agcgtgggcg ttagccgacg cagattaact tcgtattcgg gagctctgag      60

taccgtccgt gtacgcccac gctcgag                                          87


<210>  49
<211>  87
<212>  DNA
<213>  artificial sequence

<220>
<223>  Specific sequence of the oligonucleotide that recognizes the
       Estrogen Receptor protein (ER1)

<400>  49
cggaattccg agcgtgggcg ttagcccctt gtgaatgccg agagcacgat gaacaccgga      60

atccgtagcc gtacgcccac gctcgag                                          87


<210>  50
<211>  87
<212>  DNA
<213>  artificial sequence

<220>
<223>  Specific sequence of the oligonucleotide that recognizes the
       Estrogen Receptor protein (ER1)

<400>  50
cggaattccg agcgtgggcg ttagccatgt atttaagttc cgcttttttt attggacgca      60

acctatccgc ctacgcccac gctcgag                                          87


<210>  51
<211>  87
<212>  DNA
<213>  artificial sequence

<220>
<223>  Specific sequence of the oligonucleotide that recognizes the
       Estrogen Receptor protein (ER2)

<400>  51
cggaattccg agcgtgggcg ttcggactag aacgcgagga agtacggatt atcctcattt      60

gaggtaacct gtacgcccac gctcgag                                          87


<210>  52
<211>  87
<212>  DNA
<213>  artificial sequence

<220>

<223>  Specific sequence of the oligonucleotide that recognizes the
       Estrogen Receptor protein (ER2)

<400>  52
cggaattccg agcgtgggcg ttggtcccac gtcccgaatc tcgtcggcgc aagggtaagt      60

gttcgttacc gtacgcccac gctcgag                                          87


<210>  53
<211>  87
<212>  DNA
<213>  artificial sequence

<220>
<223>  Specific sequence of the oligonucleotide that recognizes the
       300-400 aa peptide fragment of HIV-gp120 protein

<400>  53
cggaattccg agcgtgggcg ttgtgccagt gcgcaaccac gcgaggtagg tcaggcgagt      60

gttcatcgtg gtacgcccac gctcgag                                          87


<210>  54
<211>  87
<212>  DNA
<213>  artificial sequence

<220>
<223>  Specific sequence of the oligonucleotide that recognizes the
       300-400 aa peptide fragment of HIV-gp120 protein

<400>  54
cggaattccg agcgtgggcg ttctcacgcc ctggagttga atttgcgctc gataaaatca      60

ttcttcagcg gtacgcccac gctcgag                                          87


<210>  55
<211>  87
<212>  DNA
<213>  artificial sequence

<220>
<223>  Specific sequence of the oligonucleotide that recognizes the
       300-400 aa peptide fragment of HIV-gp120 protein

<400>  55
cggaattccg agcgtgggcg taagtcccac gggatcgatc tgcgcttaag tatacctcaa      60

cacgcactgt gtacgcccac gctcgag                                          87


<210>  56
<211>  91
<212>  DNA
<213>  artificial sequence

<220>
<223>  Specific sequence of the oligonucleotide that recognizes the

subunit E protein of the vacuolar proton pump (VH+E)

<400>  56
tgcaggccgc ggggaaggag tggatacacg gggctgcttg atgatctggt aaagcggttt      60

gcatcgcggg ggtccttccc cgcggcctgc a                                     91


<210>  57
<211>  121
<212>  DNA
<213>  artificial sequence

<220>
<223>  Specific sequence of the oligonucleotide that recognizes the
       Huntingtin protein, amino-terminal region

<400>  57
tccattcaca gaggttttga gcgccgtgaa cgatggcagc caactagaga gagtacacgt      60

taccggtacc ccttacagac tagcgagcgg agcgaacacc tgtatgatag aggcgtgtgt     120

c                                                                     121


<210>  58
<211>  118
<212>  DNA
<213>  artificial sequence

<220>
<223>  Specific sequence of the oligonucleotide that recognizes the
       Huntingtin region, amino-terminal region

<400>  58
tccattcaca gaggttttga gccgacaaat aatacactgc aaggaagtgc tacaactacg      60

aaaagccagc agccgcggct gacggaagac ctaagctgat atgatagagg cgtgtgtc       118


<210>  59
<211>  116
<212>  DNA
<213>  artificial sequence

<220>
<223>  Specific sequence of the oligonucleotide that recognizes the
       Huntingtin region, amino-terminal region

<400>  59
tccattcaca gaggttttga gcacgatcgc taccaccgca agaatcatga gccctttcaa      60

ggtttgggac atttataacg gagatcaagt gaactgatat gatagaggcg tgtgtc         116


<210>  60
<211>  119
<212>  DNA
<213>  artificial sequence

<220>

<223>  Specific sequence of the oligonucleotide that recognizes the
       Huntingtin region, amino-terminal region

<400>  60
tccattcaca gaggttttga gcgcagaaac caaaaatacg gctcagcaaa cccccaagac      60

aaatcgagcc aacaacagta acgataatgc ccaaaccatg tatgatagag gcgtgtgtc      119


<210>  61
<211>  121
<212>  DNA
<213>  artificial sequence

<220>
<223>  Specific sequence of the oligonucleotide that recognizes the
       Huntingtin region, amino-terminal region

<400>  61
tccattcaca gaggttttga gcgcggaccg aaagcgagta actaaggacg taagcgaccg      60

ggacaacgac caactgacca cacaacctaa cccaaagcat gatatgatag aggcgtgtgt     120

c                                                                     121


<210>  62
<211>  120
<212>  DNA
<213>  artificial sequence

<220>
<223>  Specific sequence of the oligonucleotide that recognizes the
       Huntingtin region, amino-terminal region

<400>  62
tccattcaca gaggttttga gcaaagttcc caacagagct tagaatcaac gcgagaaaaa      60

acccacaaca agcatcccac catacagcac tcactctatt gtatgataga ggcgtgtgtc     120

Having described and presented the nature and scope of the invention and the embodiments of the invention, the exclusive right and property right of the invention are hereby claimed:

## Claims

1. SELECTION PROCEDURE IMPLEMENTED BY EXCESS LIGANDS based on the principle of mass action in solution **characterized in that** its products are made up of synthetic strains of oligonucleotides that recognize its high specificity target-receptors.

2. SELECTION PROCEDURE IMPLEMENTED BY EXCESS LIGANDS according to Claim 1, **characterized in that** the process to obtain a mixture of ligands consists of the following steps:
• Obtaining a library of oligonucleotides with two fixed sequences of primer binding flanking a random region of nucleotides added randomly.
• Amplifying the library so that some species can attain minimum concentration.
• Selecting ligands through the presentation of the amplified library to the target selected as receptor of the interaction and partition of species bound to the targets of the free ones.
• Analyzing and sequencing the selected species of ligands.

3. SELECTION PROCEDURE IMPLEMENTED BY EXCESS LIGANDS according to Claim 2 **characterized in that** the ligands are DNA acids and the targets are proteins or protein fragments.

4. SELECTION PROCEDURE IMPLEMENTED BY EXCESS LIGANDS according to Claim 2 **characterized in that** the library is amplified through the individual synthesis of each species or by the amplification of a pre-existing mixture.

5. SELECTION PROCEDURE IMPLEMENTED BY EXCESS LIGANDS according to Claim 3 **characterized in that** the amplification of the library is performed through PCR using complementary and reverse flanking primers.

6. SELECTION PROCEDURE IMPLEMENTED BY EXCESS LIGANDS according to Claim 5 **characterized in that** primers contain modifications.

7. SELECTION PROCEDURE IMPLEMENTED BY EXCESS LIGANDS according to Claim 6 **characterized in that** these modifications consist in adding biotine, fluorescent or radioactive agents for their detection.

8. SELECTION PROCEDURE IMPLEMENTED BY EXCESS LIGANDS according to Claim 2 **characterized in that** the targets are bound to a solid matrix.

9. SELECTION PROCEDURE IMPLEMENTED BY EXCESS LIGANDS according to Claim 8 **characterized in that** the solid matrix is nitrocellulose paper or histological sections.

10. SELECTION PROCEDURE IMPLEMENTED BY EXCESS LIGANDS according to Claim 2 **characterized in that** the targets are presented in solution.

11. SELECTION PROCEDURE IMPLEMENTED BY EXCESS LIGANDS according to Claim 8 **characterized in that** the bound ligands are separated from the free ones by rinsing the membrane or the histological sections.

12. SELECTION PROCEDURE IMPLEMENTED BY EXCESS LIGANDS according to Claim 10 **characterized in that** the bound ligands are separated from the free ones using any physical methods, such as retardation gels, nitrocellulose-filter binding, liquid phase separation, affinity columns or purification with antibodies.

13. SELECTION PROCEDURE IMPLEMENTED BY EXCESS LIGANDS according to Claims 11 y 12 **characterized in that** the bound ligands are separated from their targets using any physical means.

14. SELECTION PROCEDURE IMPLEMENTED BY EXCESS LIGANDS according to Claim 13 **characterized in that** the separation of bound ligands uses high temperature, pH modification, change of solvent, salinity increase or target degradation.

15. SELECTION PROCEDURE IMPLEMENTED BY EXCESS LIGANDS according to Claim 2 **characterized in that** the selected oligonucleotides are introduced in an appropriate vector, which is cloned, purified and sequenced to obtain each ligand separately.

16. AN OLIGO-ANTIBODY obtained according to the procedure in Claim 15 **characterized in that** it consists of an oligonucleotide which specifically binds to a protein selected as target.

17. AN OLIGO-ANTIBODY according to Claim 16 **characterized in that** it can be obtained by chemical synthesis, PCR amplification or plasmid mediated.

18. AN OLIGO-ANTIBODY according to Claim 16 **characterized in that** it consists of single-strain or double-strain nucleic acids obtained according to the protocol described in Claim 2.

19. SELECTION PROCEDURE IMPLEMENTED BY EXCESS LIGANDS according to Claim 2 **characterized in that** the target is a pure protein or a complex mixture of proteins, protein fragments, proteins or peptides modified by phosphorilation, with added sugars, huntingtin, isoprenils or any biochemical modification.

20. SELECTION PROCEDURE IMPLEMENTED BY EXCESS LIGANDS according to Claim 2 **characterized in that** the oligo-antibody or the mixture of oligo-antibodies can be used to purify proteins, affinity chromatographies, cell separation or histological detections.

21. SELECTION PROCEDURE IMPLEMENTED BY EXCESS LIGANDS according to Claim 2 **characterized in that** the oligo-antibody or the mixture of oligo-antibodies is used to inhibit or activate enzymes, block protein interactions, arrange proteins for complex enzymatic process.

22. In accordance with Claim 2, the oligo-antibody or the mixture of oligoantibodies can be used to carry drugs or nanoparticles.

23. SELECTION PROCEDURE IMPLEMENTED BY EXCESS LIGANDS according to Claim 2 **characterized in that** the oligo-antibody or the mixture of oligo-antibodies can be used to form ligand polymers.

24. SELECTION PROCEDURE IMPLEMENTED BY EXCESS LIGANDS according to Claim 22 **characterized in that** the polymer is generated by one oligo-antibody or the mixture of oligo-antibodies against the same target.

25. SELECTION PROCEDURE IMPLEMENTED BY EXCESS LIGANDS according to Claim 22 **characterized in that** the polymer is generated by one oligo-antibody or the mixture of oligo-antibodies against different targets.

26. SELECTION PROCEDURE IMPLEMENTED BY EXCESS LIGANDS **characterized in that** the method to obtain a mixture of differential ligands consists of the following steps:
• Obtaining a library of oligonucleotides with two fixed primer-binding sequences flanking a variable region of nucleotides which are randomly added.
• Amplifying the library so that some species can reach a minimum concentration.
• Selecting non-differential ligands through the presentation of the amplified library with the target selected as non-desired and receiver of the interaction and partition of the species not bound to the targets of the free ones.
• Partitioning the species *not bound* to the targets of the free ones.
• Selecting differential ligands through a second selection using the library that contains the unbound ligands .
• Analyzing and sequencing the ligands and differential targets obtained.

27. SELECTION PROCEDURE IMPLEMENTED BY EXCESS LIGANDS according to Claim 26 **characterized in that** the targets are presented as bound to a solid matrix.

28. SELECTION PROCEDURE IMPLEMENTED BY EXCESS LIGANDS according to Claim 27 **characterized in that** the solid matrix is nitrocellulose paper or histological sections.

29. SELECTION PROCEDURE IMPLEMENTED BY EXCESS LIGANDS according to Claim 27 **characterized in that** the targets are presented in solution.

30. SELECTION PROCEDURE IMPLEMENTED BY EXCESS LIGANDS according to Claim 27 **characterized in that** the bound ligands are separated from unbound ones by washing or rising the membrane or histological sections.

31. SELECTION PROCEDURE IMPLEMENTED BY EXCESS LIGANDS according to Claim 26 **characterized in that** the bound ligands are separated from the unbound ones using any physical method, such as retardation gels, nitro-cellulose filter binding, liquid phase separation, affinity columns or purification with antibodies.

32. SELECTION PROCEDURE IMPLEMENTED BY EXCESS LIGANDS according to Claims 27 and 28 **characterized in that** the bound ligands are separated from their targets using any physical means.

33. SELECTION PROCEDURE IMPLEMENTED BY EXCESS LIGANDS according to Claim 32 **characterized in that** the separation of bound ligands is done through high temperature, pH change, solvent change, salinity increase or target degradation.

34. SELECTION PROCEDURE IMPLEMENTED BY EXCESS LIGANDS according to Claim 26 **characterized in that** the selected oligonucleotides are incorporated in an appropriate cloned, purified and sequenced vector to obtain each ligand separately.

35. SELECTION PROCEDURE IMPLEMENTED BY EXCESS LIGANDS according to Claims 2 and 26 **characterized in that** the selected oligonucleotides are used for the detection of differential targets in micro-arrangements of ligands.
